(19) **Europäisches Patentamt / European Patent Office / Office européen des brevets**

(11) **EP 4 517 375 B1**

(12) **EUROPEAN PATENT SPECIFICATION**

(45) Date of publication and mention of the grant of the patent:
**22.04.2026 Bulletin 2026/17**

(21) Application number: **24196682.9**

(22) Date of filing: **27.08.2024**

(51) International Patent Classification (IPC):
*G01S 7/52* (2006.01)    *G01S 15/89* (2006.01)
*A61B 8/00* (2006.01)    *A61B 8/15* (2006.01)

(52) Cooperative Patent Classification (CPC):
**G01S 7/52038; A61B 8/15; A61B 8/5207; A61B 8/54; G01S 15/8915**

(54) **ULTRASOUND DIAGNOSIS APPARATUS AND ULTRASOUND DIAGNOSIS METHOD**

ULTRASCHALLDIAGNOSEGERÄT UND ULTRASCHALLDIAGNOSEVERFAHREN

APPAREIL DE DIAGNOSTIC PAR ULTRASONS ET PROCÉDÉ DE DIAGNOSTIC PAR ULTRASONS

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **28.08.2023 JP 2023138397**

(43) Date of publication of application:
**05.03.2025 Bulletin 2025/10**

(73) Proprietor: **Canon Medical Systems Corporation Tochigi 324-0036 (JP)**

(72) Inventor: **TAKAHASHI, Hiroki Otawara-shi, 324-0036 (JP)**

(74) Representative: **Marks & Clerk LLP 15 Fetter Lane London EC4A 1BW (GB)**

(56) References cited:
- YANG XIONGBIN ET AL: "A reverse time migration-based multistep angular spectrum approach for ultrasonic imaging of specimens with irregular surfaces", ULTRASONICS, IPC SCIENCE AND TECHNOLOGY PRESS LTD. GUILDFORD, GB, vol. 108, 31 July 2020 (2020-07-31), XP086267015, ISSN: 0041-624X, [retrieved on 20200731], DOI: 10.1016/ J.ULTRAS.2020.106233
- LIN FANGLUE ET AL: "Ultrasound contrast imaging: influence of scatterer motion in multi-pulse techniques", IEEE TRANSACTIONS ON ULTRASONICS, FERROELECTRICS, AND FREQUENCY CONTROL, IEEE, USA, vol. 60, no. 10, October 2013 (2013-10-01), XP011527578, ISSN: 0885-3010, [retrieved on 20130919], DOI: 10.1109/TUFFC.2013.2797

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD

Embodiments described herein relate generally to an ultrasound diagnosis apparatus and an ultrasound diagnosis method.

BACKGROUND

In the field of measuring seismic waves, seismic wave interferometry (which may be called a Shot Profile Migration (SPM) or a wave field correlation method) is known. The seismic wave interferometry is a method by which an epicenter position or a reflection plane underground is reconstructed and by which the epicenter position or the like is estimated by performing, for example, a correlation process on a transmission wave field obtained through a forward propagation simulation and a reception wave field obtained from a backward propagation simulation.

In this situation, since seismic waves and ultrasound waves share the property of being elastic waves, it is considered possible to reconstruct an ultrasound image by applying a method of seismic wave interferometry to an image reconstruction of an ultrasound diagnosis apparatus.

For instance, X. Yang et al., "A reverse time migration-based multistep angular spectrum approach for ultrasonic imaging of specimens with irregular surfaces", Ultrasonics 108 (2020) 106233, discloses a reverse time migration method for ultrasound imaging, wherein imaging conditions are applied to obtain a forward propagation wavefield for a source signal and a backward propagation wavefield for a received signal to reconstruct an image by cross-correlating the forward and backward propagating wavefields.

Widely used in ultrasound diagnosis apparatuses is a so-called harmonic imaging technique by which a harmonic component in an echo is extracted and visualized in an image.

For instance, F. Lin et al., "Ultrasound contrast imaging: influence of scatterer motion in multi-pulse techniques",IEEE Transactions on Ultrasonics, Ferroelectrics, and Frequency Control, vol. 60, no. 1, October 2013, discloses ultrasound imaging based on second-harmonic signals by using multi-pulse techniques such as pulse inversion or phase modulation and amplitude modulation techniques.

Harmonic imaging is important technology as being capable of suppressing echo components from undesired directions. For this reason, when the seismic wave interferometry is applied to an image reconstruction of an ultrasound diagnosis apparatus, it is desirable to be able to carry out harmonic imaging.

However, when the seismic wave interferometry or the SPM is simply applied to an image reconstruction of an ultrasound diagnosis apparatus, because a harmonic component generated in a propagation process of an ultrasound wave is not taken into consideration, it is not possible to extract and visualize the harmonic component in the echo. Thus, we need to devise a scheme.

SUMMARY OF INVENTION

An ultrasound diagnosis apparatus according to at least one aspect of the present disclosure includes transmitter/receiver circuitry and processing circuitry.

The transmitter/receiver circuitry transmits an ultrasound wave to an examined subject by using a first transmission condition and receives an echo from the examined subject. The processing circuitry generates, on a basis of the first transmission condition, a second transmission condition corresponding to transmitting an ultrasound wave obtained by adding a harmonic component to the ultrasound wave, obtains a transmission wave field by forward propagating the ultrasound wave transmitted on a basis of the second transmission condition, obtains a reception wave field by backward propagating a signal based on the echo, and generates a harmonic echo component by performing a correlation analysis between the transmission wave field and the reception wave field.

As the second transmission condition, the processing circuitry may generate a transmission condition that has a frequency band of a harmonic component in contrast to the first transmission condition and that is same as the first transmission condition except for the frequency band.

The transmitter/receiver circuitry may transmit ultrasound waves in multiple sessions that are phase-modulated into a mutually same direction and to receive the echo from the examined subject with respect to each of the ultrasound waves transmitted in the multiple sessions.

The transmiter/receiver circuitry may transmit ultrasound waves in multiple sessions by using a plurality of first transmission conditions including the first transmission condition and receive a plurality of echoes including the echo.

The processing circuitry may generate a plurality of second transmission conditions including the second transmission condition and respectively corresponding to the plurality of first transmission conditions, obtain the transmission wave field with respect to each of the plurality of second transmission conditions, obtain the reception wave field with respect to each of the echoes respectively corresponding to the plurality of first transmission conditions, perform the correlation analysis between the transmission wave fields and the reception wave fields with respect to the second transmission conditions respectively corresponding to the first transmission conditions and generate the harmonic echo component on a basis of a result of the correlation analysis.

The transmitter/receiver circuitry may transmit the ultrasound wave by using a first one of the first transmission condition in a first transmission phase to receive a first echo and transmit the ultrasound wave by using a second one of the first transmission condition in a second transmission phase different from the first transmission phase by 180 degrees to receive a second echo. The processing circuitry may generate a first one of the second transmission condition on a basis of the first one of the first transmission conditionm, generate a second one of the second transmission condition on a basis of the second one of the first transmission condition, obtain a first transmission wave field on a basis of the first one of the second transmission condition, obtain a second transmission wave field on a basis of the second one of the second transmission condition, obtain a first reception wave field on a basis of the first echo and obtain a second reception wave field on a basis of the second echo. The processing circuitry may generate a first signal distribution by performing a correlation analysis between the first transmission wave field and the first reception wave field, generate a second signal distribution by performing a correlation analysis between the second transmission wave field and the second reception wave field, and generate the harmonic echo component on a basis of the first signal distribution and the second signal distribution.

The processing circuitry may generate the harmonic echo component by performing a subtracting process between the first signal distribution and the second signal distribution.

The processing circuitry may transmit ultrasound waves in multiple sessions by using a plurality of transmission conditions, receive a plurality of echoes including the echo, handle a process in which the transmitter/receiver circuitry transmits the ultrasound waves in the multiple sessions by using the plurality of transmission conditions and receives the plurality of echoes, as the transmitter/receiver circuitry transmitting a single ultrasound wave by using the first transmission condition being singular. The processing circuitry may generate the second transmission condition on a basis of the first transmission condition, obtain the transmission wave field on a basis of the second transmission condition, obtain the reception wave field on a basis of the plurality of echoes, generate the harmonic echo component by performing the correlation analysis between the transmission wave field and the reception wave field.

The processing circuitry may extract a reception signal containing a harmonic component by combining the plurality of echoes together, and obtain the reception wave field on a basis of the extracted reception signal.

The processing circuitry may obtain the transmission wave field by forward propagating the ultrasound wave while implementing a simulation method that takes a non-linear component of a medium into consideration.

The processing circuitry may obtain the reception wave field by backward propagating the signal based on the echo while implementing a simulation method that takes a non-linear component of a medium into consideration.

The processing circuitry may implement a simulation method that uses an angular spectrum method.

An ultrasound diagnosis method according to at least one aspect of the present disclosure includes: transmitting an ultrasound wave to an examined subject by using a first transmission condition and receiving an echo from the examined subject; generating, on a basis of the first transmission condition, a second transmission condition corresponding to transmitting an ultrasound wave obtained by adding a harmonic component to the ultrasound wave; obtaining a transmission wave field by forward propagating the ultrasound wave transmitted on a basis of the second transmission condition; obtaining a reception wave field by backward propagating a signal based on the echo; and generating a harmonic echo component by performing a correlation analysis between the transmission wave field and the reception wave field.

BRIEF DESCRIPTION OF THE DRAWINGS

FIG. 1 is a diagram illustrating an exemplary configuration of an ultrasound diagnosis apparatus according to a first embodiment;

FIG. 2 is a chart for explaining a process performed by an ultrasound diagnosis apparatus 10 according to the first embodiment;

FIG. 3 is a flowchart for explaining a flow in the process performed by the ultrasound diagnosis apparatus according to the first embodiment;

FIG. 4 is a chart for explaining a process performed by the ultrasound diagnosis apparatus according to the first embodiment;

FIG. 5 is a flowchart for explaining a flow in the process performed by the ultrasound diagnosis apparatus according to the first embodiment;

FIG. 6 is a flowchart for explaining a flow in a process performed by an ultrasound diagnosis apparatus according to a second embodiment;

FIG. 7 is a chart for explaining the process performed by the ultrasound diagnosis apparatus according to the second embodiment;

FIG. 8 is another chart for explaining the process performed by the ultrasound diagnosis apparatus according to the second embodiment;

FIG. 9 is a flowchart for explaining a flow in a process performed by an ultrasound diagnosis apparatus according to a modification example of the second embodiment;

FIG. 10 is a chart for explaining a process performed by the ultrasound diagnosis apparatus according to the modification example of the second embodiment; and

FIG. 11 is a flowchart for explaining a flow in a process performed by an ultrasound diagnosis apparatus according to a third embodiment.

## DETAILED DESCRIPTION

First Embodiment

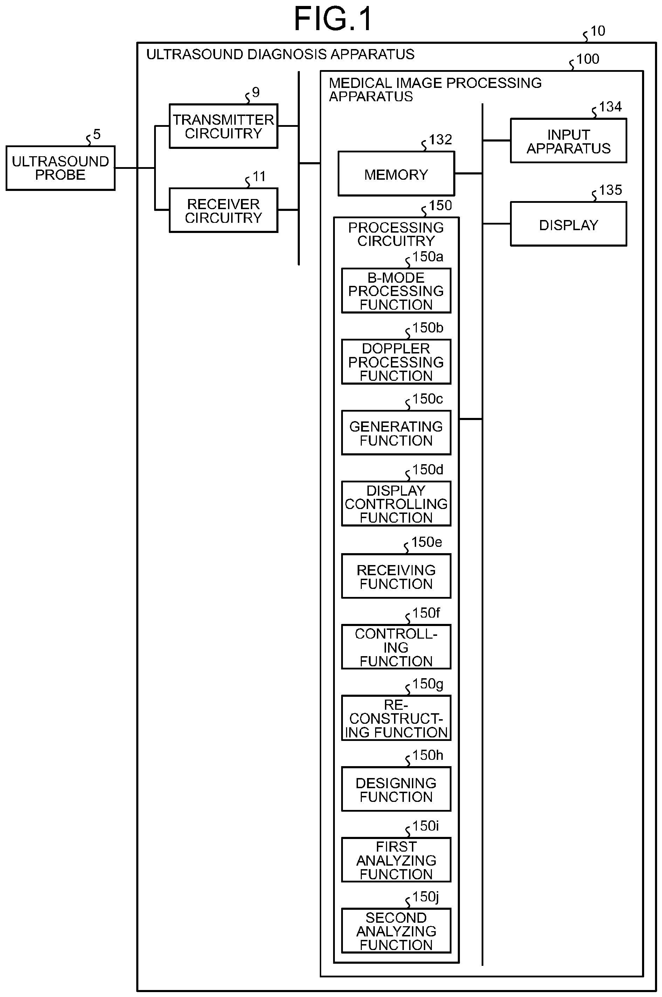

To begin with, a configuration of an ultrasound diagnosis apparatus according to a first embodiment will be explained. FIG. 1 is a block diagram illustrating an exemplary configuration of the ultrasound diagnosis apparatus according to the first embodiment. As illustrated in FIG. 1, the ultrasound diagnosis apparatus according to the first embodiment includes an ultrasound probe 5 and an ultrasound diagnosis apparatus 10. The ultrasound diagnosis apparatus 10 includes transmitter circuitry 9, receiver circuitry 11, and a medical image processing apparatus 100.

The ultrasound probe 5 includes a plurality of piezoelectric transducer elements. The plurality of piezoelectric transducer elements are configured to generate an ultrasound wave on the basis of a drive signal supplied from the transmitter circuitry 9 (explained later) included in the ultrasound diagnosis apparatus 10. Further, the plurality of piezoelectric transducer elements included in the ultrasound probe 5 are configured to receive a reflected wave from an examined subject (hereinafter, "patient") P and convert the received reflected wave into an electrical signal (a reflected-wave signal). Further, the ultrasound probe 5 includes a matching layer provided for the piezoelectric transducer elements, a backing member configured to prevent the ultrasound wave from propagating rearward from the piezoelectric transducer elements, and the like. In this situation, the ultrasound probe 5 is detachably connected to the ultrasound diagnosis apparatus 10.

When the ultrasound wave is transmitted from the ultrasound probe 5 to the patient P, the transmitted ultrasound wave is repeatedly reflected on a surface of discontinuity of acoustic impedances at a tissue in the body of the patient P, received as the reflected wave by the plurality of piezoelectric transducer elements included in the ultrasound probe 5, and converted into the reflected-wave signal. The amplitude of the reflected-wave signal is dependent on the difference between the acoustic impedances on the surface of discontinuity on which the ultrasound wave is reflected. When a transmitted ultrasound pulse is reflected on the surface of a moving blood flow, a cardiac wall, or the like, the reflected-wave signal is, due to the Doppler effect, subject to a frequency shift, depending on a velocity component of the moving members with respect to the ultrasound wave transmission direction.

It should be noted that the embodiment is applicable when the ultrasound probe 5 is a one-dimensional (1D) array probe configured to two-dimensionally scan the patient P or is a mechanical four-dimensional (4D) probe or a two-dimensional (2D) array probe configured to three-dimensionally scan the patient P.

The ultrasound diagnosis apparatus 10 is an apparatus configured to generate ultrasound image data on the basis of the reflected-wave signal received from the ultrasound probe 5. The ultrasound diagnosis apparatus 10 illustrated in FIG. 1 is capable of generating two-dimensional ultrasound image data on the basis of a two-dimensional reflected-wave signal and capable of generating three-dimensional ultrasound image data on the basis of a three-dimensional reflected-wave signal. However, the embodiment is also applicable when the ultrasound diagnosis apparatus 10 is an apparatus exclusively for two-dimensional data.

As illustrated in FIG. 1, the ultrasound diagnosis apparatus 10 includes the transmitter circuitry 9, the receiver circuitry 11, and the medical image processing apparatus 100.

The transmitter circuitry 9 and the receiver circuitry 11 are configured to control the ultrasound wave transmission/reception performed by the ultrasound probe 5 on the basis of an instruction from processing circuitry 150 including a controlling function 150f (explained later). The transmitter circuitry 9 includes a pulse generator, a transmission delay unit, a pulser, and the like and is configured to supply the drive signal to the ultrasound probe 5. The pulse generator is configured to repeatedly generate a rate pulse used for forming a transmission ultrasound wave at a prescribed Pulse Repetition Frequency (PRF). Further, the transmission delay unit is configured to apply, to the rate pulses generated by the pulse generator, delay periods that respectively correspond to the piezoelectric transducer elements and are necessary for converging the ultrasound wave generated by the ultrasound probe 5 into the form of a beam and determining transmission directionality. Further, the pulser is configured to apply the drive signal (a drive pulse) to the ultrasound probe 5, with timing based on the rate pulses.

In other words, the transmission delay unit is configured to arbitrarily adjust transmission directions of the ultrasound wave transmitted from surfaces of the piezoelectric transducer elements, by varying the delay periods applied to the rate pulses. Also, the transmission delay unit is configured to control the position of a convergence point (a transmission focus) in the depth direction of the ultrasound wave transmission, by varying the delay periods applied to the rate pulses.

Further, the transmitter circuitry 9 has a function capable of instantaneously changing a transmission frequency, transmission drive voltage, and the like, for executing a predetermined scan sequence on the basis of an instruction from the processing circuitry 150 (explained later). In particular, the capability to change the transmission drive voltage is realized by transmitter circuitry of a linear amplifier type capable of instantaneously switching the value thereof or a mechanism configured to electrically switch between a plurality of power source units.

Further, the receiver circuitry 11 includes amplifier circuitry, an Analog/Digital (A/D) converter, reception delay circuitry, an adder, quadrature detection circuitry, and the like and is configured to generate a reception signal (reflected-wave data) by performing various types of processes on the reflected-wave signals received from the ultrasound probe 5. The amplifier circuitry is configured to amplify the reflected-wave signal with respect to each channel and to perform a gain correction process. The A/D converter is configured to perform an A/D conversion on the gain-corrected reflected-wave signals. The reception delay circuitry is configured to apply, to the digital data, a reception delay period necessary for determining reception directionality. The adder is configured to perform a process of adding together the reflected-wave signals to which the reception delay period was applied by the reception delay circuitry. As a result of the adding process by the adder, reflected components from a direction corresponding to the reception directionality of the reflected-wave signals are emphasized. Further, the quadrature detection circuitry is configured to convert an output signal from the adder into an In-phase signal (an I signal) and a Quadrature-phase signal (a Q signal) in a baseband. After that, the quadrature detection circuitry is configured to transmit the I signal and the Q signal (hereinafter, "IQ signals") to the processing circuitry 150, as the reception signal (the reflected-wave data). Alternatively, the quadrature detection circuitry may convert the output signal from the adder into a Radio Frequency (RF) signal, which is then transmitted to the processing circuitry 150. The IQ signals and the RF signal serve as reception signals including phase information.

When scanning a two-dimensional region in the patient P, the transmitter circuitry 9 is configured to cause an ultrasound beam for scanning the two-dimensional region to be transmitted from the ultrasound probe 5. Further, the receiver circuitry 11 is configured to generate a two-dimensional reception signal from a two-dimensional reflected-wave signal received from the ultrasound probe 5. In contrast, when scanning a three-dimensional region in the patient P, the transmitter circuitry 9 is configured to cause an ultrasound beam for scanning the three-dimensional region to be transmitted from the ultrasound probe 5. Further, the receiver circuitry 11 is configured to generate a three-dimensional reception signal from a three-dimensional reflected-wave signal received from the ultrasound probe 5. The receiver circuitry 11 is configured to generate a reception signal on the basis of the reflected-wave signal and to transmit the generated reception signal to the processing circuitry 150.

The transmitter circuitry 9 is configured to cause the ultrasound probe 5 to transmit the ultrasound beam from a predetermined transmission position (a transmission scanning line). The receiver circuitry 11 is configured to receive, from the ultrasound probe 5, a signal defined by the reflected wave of the ultrasound beam transmitted by the transmitter circuitry 9, in a predetermined reception position (a reception scanning line). In the situation where a parallel simultaneous reception is not performed, the transmission scanning line and the reception scanning line correspond to the same scanning line. On the contrary, in the situation where a parallel simultaneous reception is performed, when the transmitter circuitry 9 causes the ultrasound probe 5 to transmit an ultrasound beam for one session on a single transmission scanning line, the receiver circuitry 11 is configured to simultaneously receive, via the ultrasound probe 5, a signal defined by the reflected wave derived from the ultrasound beam which the transmitter circuitry 9 caused the ultrasound probe 5 to transmit, in a plurality of predetermined reception positions (reception scanning lines) as a plurality of reception beams.

The medical image processing apparatus 100 is connected to the transmitter circuitry 9 and the receiver circuitry 11 and is configured to process signals received from the receiver circuitry 11 and to exercise control over the transmitter circuitry 9. The medical image processing apparatus 100 includes the processing circuitry 150, a memory 132, an input apparatus 134, and a display 135. The processing circuitry 150 includes a B-mode processing function 150a, a Doppler processing function 150b, a generating function 150c, a display controlling function 150d, a receiving function 150e, the controlling function 150f, a reconstructing function 150g, a designing function 150h, a first analyzing function 150i, and a second analyzing function 150j.

In the embodiment, processing functions executed by the B-mode processing function 150a, the Doppler processing function 150b, the generating function 150c, the display controlling function 150d, the receiving function 150e, the controlling function 150f, the reconstructing function 150g, the designing function 150h, the first analyzing function 150i, and the second analyzing function 150j are stored in the memory 132 in the form of computer-executable programs. The processing circuitry 150 is one or more processors configured to realize the functions corresponding to the programs by reading and executing the programs from the memory 132. In other words, the processing circuitry 150 that has read the programs has the functions illustrated within the processing circuitry 150 in FIG. 1. Further, although an example will be explained with reference to FIG. 1 in which the functions of the processing circuitry 150 are realized by using the single piece of processing circuitry, it is also acceptable to structure the processing circuitry 150 by combining together a plurality of independent processors, so that the functions are realized as a result of the processors executing the programs. In other words, each of the abovementioned functions may be structured as a program, so that a single piece of processing circuitry executes the program. Alternatively, a single piece of processing circuitry may realize two or more of the functions included in the processing circuitry 150. In another example, one or more specific functions may be installed in dedicated and independent program executing circuitry.

Further, in FIG. 1, the processing circuitry 150, the B-mode processing function 150a, the Doppler processing function 150b, the generating function 150c, the display controlling function 150d, the receiving function 150e, the controlling function 150f, the reconstructing function 150g, the designing function 150h, the first analyzing function 150i, and the second analyzing function 150j are examples of a B-mode processing unit, a Doppler processing unit, a generating unit, a display controlling unit, a receiving unit, a controlling unit, a reconstructing unit, a designing unit, a first analyzing unit, and a second analyzing unit, respectively. Further, the transmitter circuitry 9 and the receiver circuitry 11 structure an example of a transmission/reception unit.

The term "processor" used in the above explanations denotes, for example, a Central Processing Unit (CPU), a Graphical Processing Unit (GPU), or circuitry such as an Application Specific Integrated Circuit (ASIC) or a programmable logic device (e.g., a Simple Programmable Logic Device (SPLD), a Complex Programmable Logic Device (CPLD), or a Field Programmable Gate Array (FPGA)). The one or more processors are configured to realize the functions by reading and executing the programs saved in the memory 132.

Further, instead of having the programs saved in the memory 132, it is also acceptable to directly incorporate the programs in the circuitry of the one or more processors. In that situation, the one or more processors are configured to realize the functions by reading and executing the programs incorporated in the circuitry thereof. Further, the transmitter circuitry 9, the receiver circuitry 11, and the like built in the ultrasound diagnosis apparatus 10 may be configured by using hardware such as integrated circuitry or may be a program modularized in the form of software.

The processing circuitry 150 is a processing unit configured to perform various types of signal processing processes on the reception signal received from the receiver circuitry 11. The processing circuitry 150 includes the B-mode processing function 150a, the Doppler processing function 150b, the generating function 150c, the display controlling function 150d, the receiving function 150e, the controlling function 150f, the reconstructing function 150g, the designing function 150h, the first analyzing function 150i, and the second analyzing function 150j.

By employing the B-mode processing function 150a, the processing circuitry 150 is configured to receive data from the receiver circuitry 11 and to generate data (B-mode data) in which signal intensities are expressed as levels of brightness, by performing a logarithmic amplifying process, an envelope detecting process, a logarithmic compressing process, and/or the like.

Further, by employing the Doppler processing function 150b, the processing circuitry 150 is configured to generate data (Doppler data) obtained by extracting moving member information such as velocity, dispersion, power, and the like caused by the Doppler effect with respect to multiple points, by performing a frequency analysis to obtain velocity information from the reception signal (the reflected-wave data) received from the receiver circuitry 11.

The B-mode processing function 150a and the Doppler processing function 150b illustrated in FIG. 1 are capable of processing both two-dimensional reflected-wave data and three-dimensional reflected-wave data.

By employing the display controlling function 150d, the processing circuitry 150 is configured to exercise control so as to cause the display 135 to display display-purpose ultrasound image data stored in the memory 132.

By employing the receiving function 150e, the processing circuitry 150 is configured to receive various types of operations from a user, via the input apparatus 134.

By employing the controlling function 150f, the processing circuitry 150 is configured to control all the processes performed by the ultrasound diagnosis apparatus. More specifically, by employing the controlling function 150f, the processing circuitry 150 is configured to control processes performed by the transmitter circuitry 9, the receiver circuitry 11, and the processing circuitry 150, on the basis of various types of setting requests input by an operator via the input apparatus 134 and various types of control programs and various types of data read from the memory 132.

By employing the generating function 150c, the processing circuitry 150 is configured to generate the ultrasound image data from the data generated by the B-mode processing function 150a and the Doppler processing function 150b. By employing the generating function 150c, the processing circuitry 150 is configured to generate two-dimensional B-mode image data in which intensities of the reflected wave are expressed with brightness levels, from two-dimensional B-mode data generated by the B-mode processing function 150a. Further, by employing the generating function 150c, the processing circuitry 150 is configured to generate two-dimensional Doppler image data expressing the moving member information, from two-dimensional Doppler data generated by the Doppler processing function 150b. The two-dimensional Doppler image data may be velocity image data, dispersion image data, power image data, or image data combining these types of data.

Further, by employing the generating function 150c, the processing circuitry 150 is configured to generate the display-purpose ultrasound image data, by converting (by performing a scan convert process) a scanning line signal sequence from an ultrasound scan into a scanning line signal sequence in a video format used by television, for example. Further, as various types of image processing processes besides the scan convert process, the processing circuitry 150 is configured, by employing the generating function 150c, to perform, for example, an image processing process (a smoothing process) to re-generate an average brightness value image, an image processing process (an edge enhancement process) that uses a differential filter inside an image, or the like, by using a plurality of image frames resulting from the scan convert process. Also, by employing the generating function 150c, the processing circuitry 150 is configured to perform various types of rendering processes on volume data, to generate two-dimensional image data for displaying the volume data on the display 135.

Further, the processing circuitry 150 includes the reconstructing function 150g, the designing function 150h, the first analyzing function 150i, and the second analyzing function 150j. These functions will be explained later.

The memory 132 is configured by using a semiconductor memory element such as a Random Access Memory (RAM) or a flash memory, or a hard disk, an optical disc, or the like. The memory 132 is a memory configured to store therein data such as display-purpose image data generated by the processing circuitry 150. Further, the memory 132 is also capable of storing therein data generated by the B-mode processing function 150a or the Doppler processing function 150b. After a diagnosing process, for example, the operator is able to invoke any of the B-mode data and the Doppler data stored in the memory 132, so that the invoked data serves as the display-purpose ultrasound image data after being routed through the processing circuitry 150. Further, the memory 132 is also capable of storing therein the reception signal (the reflected-wave data) output by the receiver circuitry 11.

In addition, the memory 132 is configured to store therein, as necessary, control programs for performing the ultrasound wave transmission/reception, image processing processes, and display processes, as well as diagnosis information (e.g., a patient ID, a medical doctor's observations, etc.) and various types of data such as diagnosis protocols and various types of body marks.

The input apparatus 134 is configured to receive various types of instructions and inputs of information from the operator. For example, the input apparatus 134 may be pointing equipment such as a mouse or a trackball, selecting equipment such as a mode changing switch, and/or input equipment such as a keyboard.

Under control of the controlling function 150f and the like, the display 135 is configured to display, among others, a Graphical User Interface (GUI) used for receiving inputs of image taking conditions, images generated by the generating function 150c or the like. For example, the display 135 is display equipment such as a liquid display monitor. The display 135 is an example of a display unit. The display 135 includes a mouse, a keyboard, a button, a panel switch, a touch command screen, a foot switch, a trackball, a joystick, and/or the like.

Next, a background of the embodiment will be explained.

In the field of measuring seismic waves, seismic wave interferometry (which may be called a Shot Profile Migration (SPM) or a wave field correlation method) is known. The seismic wave interferometry is a method by which an epicenter position or a reflection plane underground is reconstructed and by which the epicenter position or the like is estimated by performing, for example, a correlation process on a transmission wave field obtained through a forward propagation simulation and a reception wave field obtained through a backward propagation simulation.

In this situation, since seismic waves and ultrasound waves share the property of being elastic waves, it is considered possible to reconstruct an ultrasound image by applying a method of seismic wave interferometry to an image reconstruction of an ultrasound diagnosis apparatus.

Widely used in ultrasound diagnosis apparatuses is a so-called harmonic imaging technique by which a harmonic component in an echo is extracted and visualized in an image. Harmonic imaging is important technology as being capable of suppressing echo components from undesired directions. For this reason, when the seismic wave interferometry is applied to an image reconstruction of an ultrasound diagnosis apparatus, it is desirable to be able to carry out harmonic imaging.

However, when the seismic wave interferometry or the SPM is simply applied to an image reconstruction of an ultrasound diagnosis apparatus, because a harmonic component generated in a propagation process of an ultrasound wave is not taken into consideration, it is not possible to extract and visualize the harmonic component in the echo. Thus, we need to devise a scheme. Non-Patent Literature 1 proposes an ultrasound image reconstructing method to which seismic wave interferometry is applied. However, a transmission sound field is obtained through a forward propagation simulation, on the basis of a transmission signal from piezoelectric transducer elements, which means that a harmonic component generated in the process of the transmission signal propagating through a living body is not taken into consideration.

The ultrasound diagnosis apparatus of the embodiment is based on the background described above. The ultrasound diagnosis apparatus according to the embodiment includes the transmission/reception unit and the processing circuitry 150. The transmission/reception unit is configured to transmit an ultrasound wave to a patient by using a first transmission condition and to receive an echo from the patient. By employing the designing function 150h, the processing circuitry 150 is configured, on the basis of the first transmission condition, to generate a second transmission condition corresponding to transmitting an ultrasound wave obtained by adding a harmonic component to the ultrasound wave. By employing the first analyzing function 150i, the processing circuitry 150 is configured to obtain a transmission wave field by forward propagating the ultrasound wave transmitted on the basis of the second transmission condition. By employing the second analyzing function 150j, the processing circuitry 150 is configured to obtain a reception wave field by backward propagating a signal based on the echo. By employing the reconstructing function 150g, the processing circuitry 150 is configured to generate a harmonic echo component by performing a correlation analysis between the transmission wave field and the reception wave field.

Further, an ultrasound diagnosis method according to the embodiment includes: transmitting an ultrasound wave to a patient by using a first transmission condition and receiving an echo from the patient; generating, on the basis of the first transmission condition, a second transmission condition corresponding to transmitting an ultrasound wave obtained by adding a harmonic component to the ultrasound wave; obtaining a transmission wave field by forward propagating the ultrasound wave transmitted on the basis of the second transmission condition; obtaining a reception wave field by backward propagating a signal based on the echo; and generating a harmonic echo component by performing a correlation analysis between the transmission wave field and the reception wave field.

As explained above, it is possible to apply the seismic wave interferometry to the harmonic imaging of the ultrasound diagnosis apparatus. For example, it is possible to reconstruct an ultrasound image, while keeping a high capability to suppress echo components from undesired directions, to an extent equal to or higher than capabilities of beam forming technology.

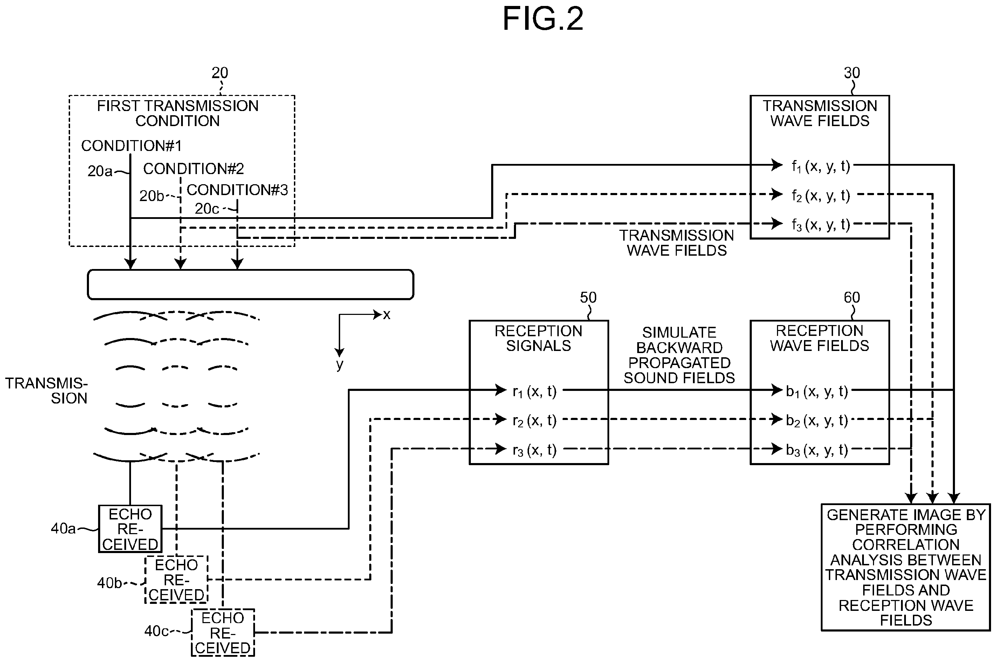

Processes performed by the ultrasound diagnosis apparatus 10 according to the first embodiment will be explained, with reference to FIGS. 2 to 5.

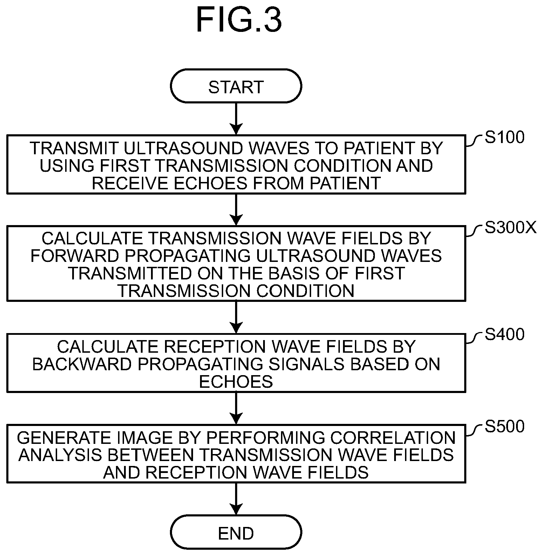

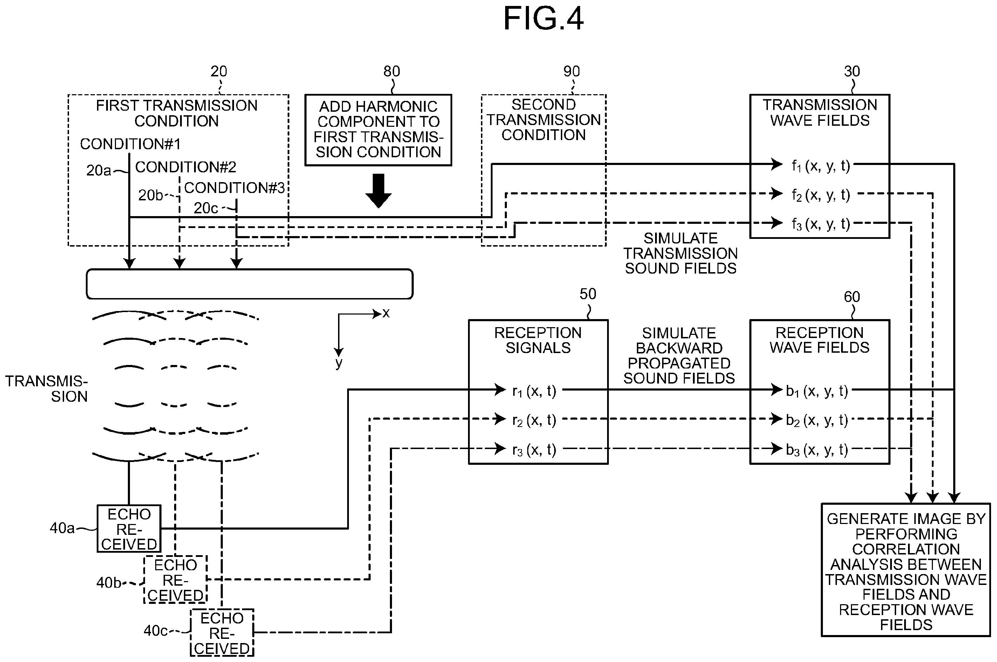

At first, an embodiment in which seismic wave interferometry is simply applied to an ultrasound diagnosing process will be explained, with reference to FIGS. 2 and 3. FIG. 2 is a schematic chart for explaining a process performed when the seismic interferometry is simply applied to an ultrasound diagnosis apparatus. FIG. 3 is a flowchart indicating the process performed by the ultrasound diagnosis apparatus 10 when the seismic interferometry is simply applied to the ultrasound diagnosis apparatus. The flowchart illustrated in FIG. 2 is different from the flowchart in FIG. 4 illustrating processes for performing harmonic imaging in that the process at step S200 (explained later) is not performed and that step S300X is performed in FIG. 2 in place of step S300 in FIG. 4.

To begin with, at step S100, the transmitter circuitry 9 and the receiver circuitry 11 serving as the transmission/reception unit transmit an ultrasound wave to a patient by using the first transmission condition and receive an echo from the patient. Typically, the ultrasound diagnosis apparatus 10 employs the transmitter circuitry 9 to transmit ultrasound waves in multiple sessions into mutually-different directions, so that the receiver circuitry 11 receives an echo from the patient with respect to each of the ultrasound waves transmitted in the multiple sessions. For example, as schematically illustrated in FIG. 2, the transmitter circuitry 9 transmits ultrasound waves 20a, 20b, and 20c in multiple sessions. Further, with respect to the ultrasound waves 20a, 20b, and 20c transmitted in the multiple sessions, the receiver circuitry 11 receives echoes 40a, 40b, and 40c from the patient, respectively, and thus acquires, as reception signals 50, reception signals $r_1(x,t)$, $r_2(x,t)$, and $r_3(x,t)$ where x denotes a position coordinate of the ultrasound beam in the advancement direction, whereas t denotes a time.

In this situation, the first transmission condition denotes, typically, a transmission condition such as an amplitude, a phase, a frequency, a transmission aperture, or transmission apodization of the ultrasound wave to be transmitted. In this situation, for example, the transmitter circuitry 9 transmits the ultrasound wave 20a by using transmission condition #1, transmits the ultrasound wave 20b by using transmission condition #2, and transmits the ultrasound wave 20c by using transmission condition #3. In this situation, transmission condition #1, transmission condition #2, and transmission condition #3 are collectively regarded as a single transmission condition and will be handled as a first transmission condition 20.

Subsequently, at step S300X, by employing the first analyzing function 150i, the processing circuitry 150 calculates transmission wave fields 30, by forward propagating the ultrasound waves transmitted on the basis of the first transmission condition, through a forward propagation simulation. In an example, by employing the first analyzing function 150i, the processing circuitry 150 may be configured to calculate transmission wave fields $f_1(x,y,t)$, $f_2(x,y,t)$, and $f_3(x,y,t)$ by forward propagating the ultrasound wave 20a, the ultrasound wave 20b, and the ultrasound wave 20c transmitted by using transmission condition #1, transmission condition #3, and transmission condition #3, through the forward propagation simulation. In this situation, x denotes a position coordinate of the ultrasound beam in a direction perpendicular to the advancement direction; y denotes a position coordinate of the ultrasound beam in the advancement direction; and t denotes a time. By employing the first analyzing function 150i, the processing circuitry 150 may be configured to calculate the transmission wave fields 30, on the basis of the first transmission condition, by simulating time development of a wave equation, for instance. In an example, by employing the first analyzing function 150i, the processing circuitry 150 may be configured to calculate the transmission wave fields 30 on the basis of the first transmission condition, by implementing a Finite Difference Time Domain (FDTD) method, for instance. In another example, by employing the first analyzing function 150i, the processing circuitry 150 may be configured to calculate the transmission wave fields 30 on the basis of the first transmission condition, by implementing a simulation method using an angular spectrum method, for instance.

After that, at step S400, by employing the second analyzing function 150j, the processing circuitry 150 calculates reception wave fields 60 by backward propagating the reception signals 50 based on the echoes acquired at step S100. In an example, by employing the second analyzing function 150j, the processing circuitry 150 may be configured to calculate reception wave fields $b_1(x,y,t)$, $b_2(x,y,t)$, and $b_3(x,y,t)$ from the reception signals $r_1(x)$, $r_2(x)$ and $r_3(x)$, respectively, by simulating time development of a wave equation while implementing the FDTD method, for instance.

Subsequently, at step S500, by employing the reconstructing function 150g, the processing circuitry 150 generates an image by performing a correlation analysis between the transmission wave fields and the reception wave fields. More specifically, an image I is generated by using Expression (1) presented below.

$$I(x,y) = \sum_{n=1}^{N} \int f_n^*(x,y,t) b_n(x,y,t)\, dt \qquad \cdots (1)$$

In Expression (1), n denotes an index for distinguishing the ultrasound waves 20a, 20b, 20c, and so on from one another. N denotes the number of sessions of ultrasound wave transmissions included in a series of ultrasound wave transmissions. The symbol "*" denotes a complex conjugate.

As explained above, the processing circuitry 150 is configured to generate the image I, by implementing the SPM method. However, when performing harmonic imaging is taken into consideration, the first transmission condition under which the transmitter circuitry 9 performs the ultrasound wave transmissions is a transmission condition that usually contains no high-order harmonic component. Accordingly, the transmission wave fields 30 obtained through the forward propagation simulation unfortunately contains no high-order harmonic component, either. As a result, even when the correlation analysis is performed between the transmission wave fields 30 and the reception wave fields 60 at step S500, the high-order harmonic component is not sufficiently extracted in the image I. In other words, because the reception waves are processed by matched-filtering based on a transmission frequency band, the frequency band of the reception waves is subject to a band restriction, which suppresses a signal of the high-order harmonic component.

To cope with the circumstances described above, the ultrasound diagnosis apparatus 10 according to the embodiment is configured, on the basis of the first transmission condition, to generate a second transmission condition corresponding to transmitting an ultrasound wave obtained by adding a harmonic component to the ultrasound wave and to further perform an image generating process based thereon. In an example, by employing the designing function 150h, the processing circuitry 150 may be configured to generate the second transmission condition obtained by adding, to the first transmission condition, a pulse signal in a wide band covering a propagation harmonic component or of a band itself of the propagation harmonic component and to further perform the image generating process based thereon. With this configuration, it is possible to extract a high-order harmonic component.

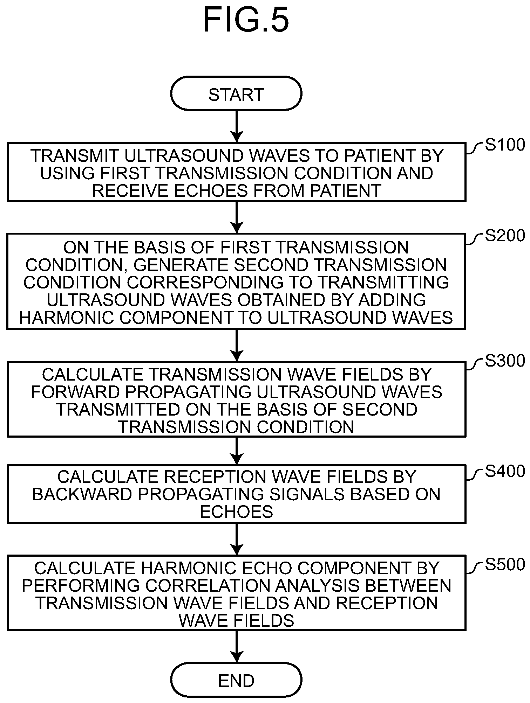

These aspects will be explained with reference to FIGS. 4 and 5. FIG. 4 is a chart for explaining a process performed by the ultrasound diagnosis apparatus 10 according to the first embodiment. FIG. 5 is a flowchart for explaining a flow in the process performed by the ultrasound diagnosis apparatus 10 according to the first embodiment.

Similarly to the example in FIG. 2, to begin with, at step S100, the transmitter circuitry 9 and the receiver circuitry 11 serving as the transmission/reception unit transmit ultrasound waves to the patient by using the first transmission condition and receive echoes from the patient. The ultrasound diagnosis apparatus 10 employs the transmitter circuitry 9 to transmit ultrasound waves in multiple sessions into mutually-different directions, for example, so that the receiver circuitry 11 receives an echo from the patient with respect to each of the ultrasound waves transmitted in the multiple sessions. For example, as schematically illustrated in FIG. 4, the transmitter circuitry 9 transmits the ultrasound waves 20a, 20b, and 20c in the multiple sessions that are phase-modulated into mutually the same direction.

Further, with respect to the ultrasound waves 20a, 20b, and 20c transmitted in the multiple sessions, the receiver circuitry 11 receives echoes 40a, 40b, and 40c from the patient, respectively, and thus acquires, as the reception signals 50, reception signals $r_1(x,t)$, $r_2(x,t)$, and $r_3(x,t)$, respectively, where x denotes a position coordinate of the ultrasound beam in the advancement direction, whereas t denotes a time.

In this situation, the first transmission condition denotes, typically, a transmission condition such as an amplitude, a phase, a frequency, a transmission aperture, or transmission apodization of the ultrasound wave to be transmitted. In this situation, for example, the transmitter circuitry 9 transmits the ultrasound wave 20a by using transmission condition #1, transmits the ultrasound wave 20b by using transmission condition #2, and transmits the ultrasound wave 20c by using transmission condition #3. Further, transmission condition #1, transmission condition #2, and transmission condition #3 are collectively regarded as a single transmission condition and will be handled as the first transmission condition 20.

Subsequently, at step S200, by employing the designing function 150h, the processing circuitry 150 generates, on the basis of the first transmission condition, a second transmission condition corresponding to transmitting an ultrasound wave obtained by adding a harmonic component to the ultrasound wave. In this situation, the harmonic component is artificially added to the first transmission condition under which the ultrasound waves were actually transmitted, for the purpose of making it possible to extract the high-order harmonic component contained in the reception wave fields, at the time of performing the correlation analysis between the transmission wave fields and the reception wave fields in the process at step S500. In this manner, by employing the designing function 150h, the processing circuitry 150 is configured to generate a second transmission condition 90 by artificially adding a harmonic component 80 to the first transmission condition 20 under which the ultrasound wave transmissions were actually performed and to thus make it possible to extract the high-order harmonic component at the time of performing the correlation analysis between the transmission wave fields and the reception wave fields.

In this situation, a first mode in which the processing circuitry 150 adds the harmonic component to the first transmission condition by employing the designing function 150h may be, for example, generating the second transmission condition 90 by adding, to the first transmission condition 20, a transmission condition of a signal in a wide band that widely covers the propagation harmonic component. Further, a second mode in which the processing circuitry 150 adds the harmonic component to the first transmission condition by employing the designing function 150h may be, for example, generating the second transmission condition 90 by adding, to the first transmission condition 20, a pulse signal that exhibits a high signal intensity only in the band itself of the propagation harmonic component. In an example, by employing the designing function 150h, the processing circuitry 150 may be configured to generate, as the second transmission condition, a transmission condition that has a frequency band of a harmonic component in contrast to the first transmission condition and that is the same as the first transmission condition except for the frequency band. In other words, by employing the designing function 150h, the processing circuitry 150 is configured to generate the second transmission condition having the different frequency from that of the first transmission condition.

However, possible embodiments are not limited to the above example. By employing the designing function 150h, the processing circuitry 150 may be configured to generate a second transmission condition in which, for example, at least one condition selected from among the following is different from that in the first transmission condition: amplitude, phase, transmission aperture, and transmission apodization. In an example, by employing the designing function 150h, the processing circuitry 150 may be configured to generate a second transmission condition for transmitting an ultrasound wave having a narrower beam than the ultrasound wave transmitted under the first transmission condition, by changing the transmission aperture or the transmission apodization from that in the first transmission condition, so as to reduce impacts of a side lobe, for example.

Further, the configuration of the processing circuitry 150 configured to employ the designing function 150h so as to generate the second transmission condition by changing a condition other than the frequency such as the amplitude, the phase, the transmission aperture, the transmission apodization, or the like, from that in the first transmission condition is similarly applicable also to the second and later embodiments and to modification examples of those embodiments.

Subsequently, at step S300, by employing the first analyzing function 150i, the processing circuitry 150 calculates the transmission wave fields 30, by forward propagating the ultrasound wave transmitted on the basis of the second transmission condition, through a forward propagation simulation. In an example, by employing the first analyzing function 150i, the processing circuitry 150 may be configured to calculate transmission wave fields $f_1(x,y,t)$, $f_2(x,y,t)$, and $f_3(x,y,t)$ by forward propagating the ultrasound waves transmitted by using the second transmission condition 90 obtained by adding the harmonic component 80 to transmission condition #1, transmission condition #2, and transmission condition #3, through the forward propagation simulation. In this situation, x denotes a position coordinate of the ultrasound beam in a direction perpendicular to the advancement direction; y denotes a position coordinate of the ultrasound beam in the advancement direction; and t denotes a time. By employing the first analyzing function 150i, the processing circuitry 150 may be configured to calculate the transmission wave fields 30, on the basis of the second transmission condition, by simulating time development of a wave equation, for instance. In an example, by employing the first analyzing function 150i, the processing circuitry 150 may be configured to calculate the transmission wave fields 30 on the basis of the second transmission condition, by implementing the FDTD method, for instance. In another example, by employing the first analyzing function 150i, the processing circuitry 150 may be configured to calculate the transmission wave fields 30 on the basis of the second transmission condition, by implementing a simulation method using an angular spectrum method, for instance.

After that, at step S400, by employing the second analyzing function 150j, the processing circuitry 150 calculates the reception wave fields 60 by backward propagating the reception signals 50 based on the echoes acquired at step S100. In an example, by employing the second analyzing function 150j, the processing circuitry 150 may be configured to calculate reception wave fields $b_1(x,y,t)$, $b_2(x,y,t)$, and $b_3(x,y,t)$ from the reception signals $r_1(x)$, $r_2(x)$ and $r_3(x)$, respectively, by simulating time development of a wave equation while implementing the FDTD method, for instance. In another example, by employing the second analyzing function 150j, the processing circuitry 150 may be configured to calculate the reception wave fields 60 by backward propagating the reception signals 50, by implementing a simulation method using an angular spectrum method.

Subsequently, at step S500, by employing the reconstructing function 150g, the processing circuitry 150 generates a harmonic echo component by performing a correlation analysis between the transmission wave fields and the reception wave fields. More specifically, by employing the reconstructing function 150g, the processing circuitry 150 generates an image I containing the harmonic echo component by using Expression (1) presented above.

As explained above, in the first embodiment, by employing the designing function 150h, the processing circuitry 150 is configured, on the basis of the first transmission condition, to generate the second transmission condition obtained by adding the harmonic component to the ultrasound wave and to further perform the correlation analysis based thereon. As a result, it is possible to extract the harmonic component and to thus enhance image quality.

Second Embodiment

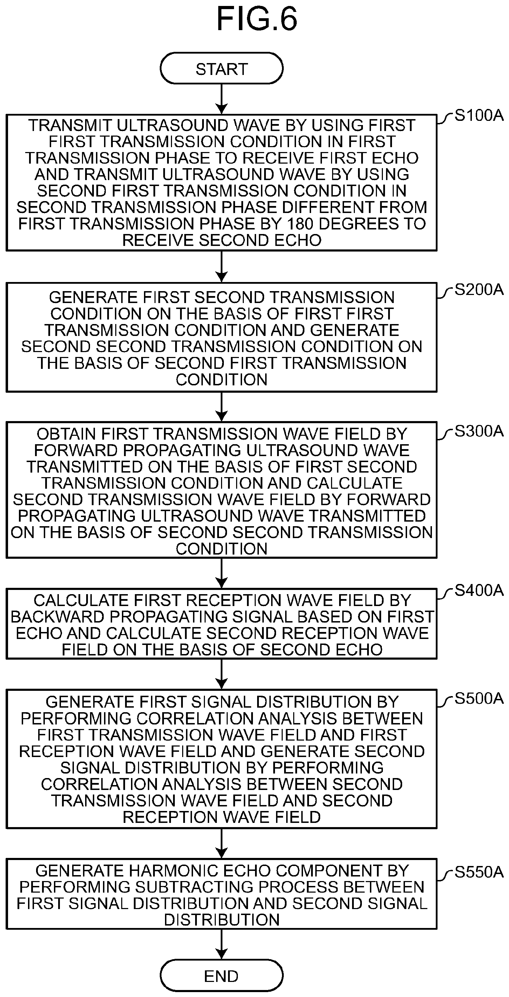

In the first embodiment, the general embodiment was explained in which, at step S100, the transmission/reception unit transmits the one or more ultrasound waves to the patient, by using the first transmission condition. In a second embodiment, an example will be explained further in detail with reference to FIGS. 6 to 8 in which the transmission/reception unit transmits a plurality of ultrasound waves by implementing a Pulse Inversion method.

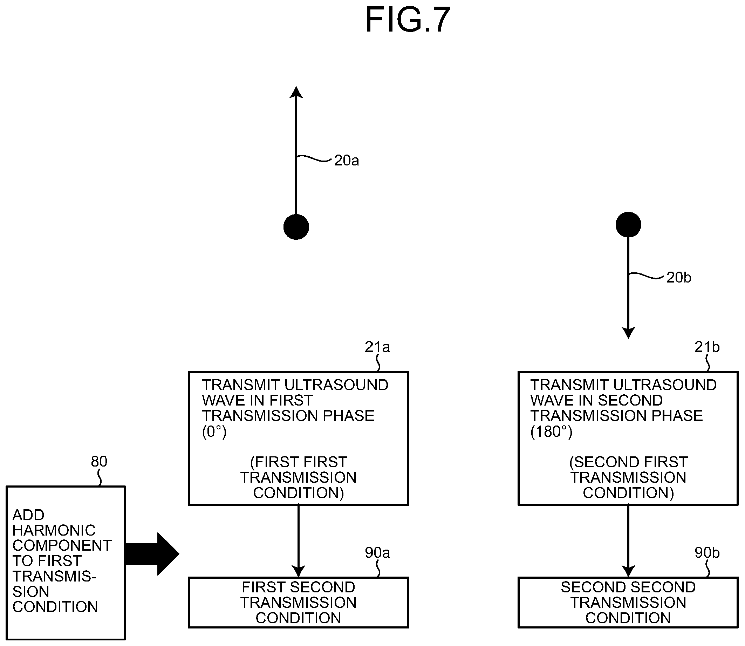

FIG. 6 is a flowchart for explaining a flow in a process performed by the ultrasound diagnosis apparatus 10 according to the second embodiment. To begin with, at step S100A, the transmitter circuitry 9 and the receiver circuitry 11 serving as the transmission/reception unit transmit ultrasound waves to a patient by using the first transmission condition and receive echoes from the patient. The ultrasound diagnosis apparatus 10 employs the transmitter circuitry 9 to transmit the ultrasound waves in multiple sessions that are phase-modulated into mutually the same direction, so that the receiver circuitry 11 receives an echo from the patient with respect to each of the ultrasound waves transmitted in the multiple sessions. In other words, the transmitter circuitry 9 is configured to transmit the ultrasound waves in the multiple sessions by performing a plurality of first transmissions, so that the receiver circuitry 11 receives the plurality of echoes. For example, when one set of ultrasound wave transmissions is performed over two transmission sessions, as illustrated in FIG. 7, the transmitter circuitry 9 transmits the ultrasound wave 20a by using a first first transmission condition (a first one of the first transmission condition) 21a by which the ultrasound wave is transmitted in a 0-degree transmission phase being a first transmission phase so that a first echo is received and transmits the ultrasound wave 20b by using a second first transmission condition (a second one of the first transmission condition) 21b by which the ultrasound wave is transmitted in a second transmission phase being a transmission phase different from the first transmission phase by 180 degrees so that a second echo is received.

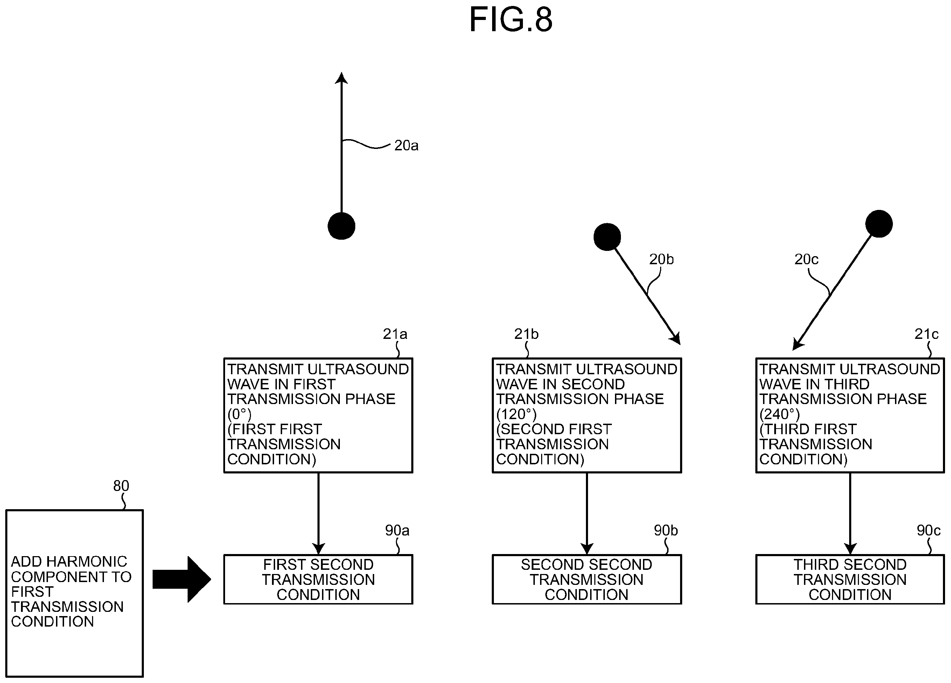

However, possible embodiments are not limited to performing one set of ultrasound wave transmissions over two transmission sessions. For example, it is also acceptable to perform one set of ultrasound wave transmissions over three transmission sessions. In that situation, as illustrated in FIG. 8, the transmitter circuitry 9 transmits the ultrasound wave 20a by using a first first transmission condition 21a by which the ultrasound wave is transmitted in a 0-degree transmission phase being a first transmission phase so that a first echo is received, also transmits the ultrasound wave 20b by using a second first transmission condition 21b by which the ultrasound wave is transmitted in a second transmission phase being a transmission phase different from the first transmission phase by 120 degrees so that a second echo is received, and transmits the ultrasound wave 20c by using a third first transmission condition 21c by which the ultrasound wave is transmitted in a third transmission phase being a transmission phase different from the first transmission phase by 240 degrees so that a third echo is received.

After that, returning to the description of FIG. 6, at step S200A, by employing the designing function 150h, the processing circuitry 150 generates, on the basis of the first transmission conditions, second transmission conditions corresponding to transmitting ultrasound waves obtained by adding a harmonic component to the ultrasound waves. In other words, by employing the designing function 150h, the processing circuitry 150 is configured to generate a plurality of second transmission conditions respectively corresponding to the plurality of first transmission conditions.

More specifically, as illustrated in FIG. 7, when the one set of ultrasound wave transmissions is performed over the two transmission sessions, by employing the designing function 150h, the processing circuitry 150 generates a first second transmission condition (a first one of the second transmission condition) 90a by adding the harmonic component 80 to the first first transmission condition 21a on the basis of the first first transmission condition 21a and also generates a second second transmission condition (a second one of the second transmission condition) 90b by adding the harmonic component 80 to the second first transmission condition 21b on the basis of the second first transmission condition 21b. Further, as illustrated in FIG. 8, when the one set of ultrasound wave transmissions is performed over the three transmission sessions, by employing the designing function 150h, the processing circuitry 150 generates a first second transmission condition 90a by adding the harmonic component 80 to the first first transmission condition 21a on the basis of the first first transmission condition 21a, also generates a second second transmission condition 90b by adding the harmonic component 80 to the second first transmission condition 21b on the basis of the second first transmission condition 21b, and generates a third second transmission condition 90c by adding the harmonic component 80 to the third first transmission condition 21c on the basis of the third first transmission condition 21c.

In this situation, a first mode in which the processing circuitry 150 adds the harmonic component to the first transmission condition by employing the designing function 150h may be, for example, generating the second transmission condition 90 by adding, to the first transmission condition 20, a transmission condition of a signal in a wide band that widely covers the propagation harmonic component. Further, a second mode in which the processing circuitry 150 adds the harmonic component to the first transmission condition by employing the designing function 150h may be, for example, generating the second transmission condition 90 by adding, to the first transmission condition 20, a pulse signal that exhibits a high signal intensity only in the band itself of the propagation harmonic component. In an example, by employing the designing function 150h, the processing circuitry 150 may be configured to generate, as the second transmission condition, a transmission condition that has a frequency band of a harmonic component in contrast to the first transmission condition and that is the same as the first transmission condition except for the frequency band. The processing circuitry 150 is configured to generate the plurality of second transmission conditions by performing the process described above on each of the plurality of first transmission conditions. By employing the designing function 150h, the processing circuitry 150 is configured to generate the second transmission conditions in each of which at least one condition selected from among the following is different from that in the first transmission condition: amplitude, phase, frequency, transmission aperture, and transmission apodization.

At step S300A, by employing the first analyzing function 150i, the processing circuitry 150 calculates the transmission wave fields 30 by forward propagating the ultrasound waves transmitted on the basis of the second transmission conditions, through a forward propagation simulation. In other words, by employing the first analyzing function 150i, the processing circuitry 150 obtains the transmission wave field with respect to each of the plurality of second transmission conditions. For example, by employing the first analyzing function 150i, the processing circuitry 150 obtains a first transmission wave field by performing a forward propagation simulation on the basis of a first second transmission condition and obtains a second transmission wave field by performing a forward propagation simulation on the basis of a second second transmission condition. By employing the first analyzing function 150i, the processing circuitry 150 may be configured to calculate the transmission wave fields 30 on the basis of the second transmission conditions 90, for example, by simulating time development of a wave equation, for example, by implementing a simulation method using the FDTD method or an angular spectrum method.

After that, at step S400A, by employing the second analyzing function 150j, the processing circuitry 150 calculates the reception wave fields 60 by backward propagating the reception signals 50 based on the echo obtained at step S100. In other words, by employing the second analyzing function 150j, the processing circuitry 150 obtains the reception wave fields 60 by performing the backward propagation simulation on the echoes respectively corresponding to the plurality of first transmission conditions. More specifically, by employing the second analyzing function 150j, the processing circuitry 150 obtains a first reception wave field on the basis of a first echo corresponding to a first first transmission conditions and obtains a second reception wave field on the basis of a second echo corresponding to a second first transmission condition. For example, by employing the second analyzing function 150j, the processing circuitry 150 may be configured to obtain the reception wave fields by backward propagating the echoes corresponding to the transmission conditions, for example, by simulating time development of a wave equation while using the FDTD method or by implementing a simulation method using an angular spectrum method.

Subsequently, at step S500A, by employing the reconstructing function 150g, the processing circuitry 150 performs a correlation analysis by evaluating the right-hand side of Expression (1), for example, between the transmission wave fields and the reception wave fields with respect to the second transmission conditions respectively corresponding to the first transmission conditions. By employing the reconstructing function 150g, the processing circuitry 150 generates a first signal distribution by performing a correlation analysis on the basis of Expression (1) between a first transmission wave field and a first reception wave field and generates a second signal distribution by performing a correlation analysis on the basis of Expression (1) between a second transmission wave field and a second reception wave field.

After that at step S550A, by employing the reconstructing function 150g, the processing circuitry 150 generates a harmonic echo component on the basis of results of the correlation analyses. For example, by employing the reconstructing function 150g, the processing circuitry 150 generates the harmonic echo component on the basis of the first signal distribution and the second signal distribution. In this situation, the transmission phase of the transmission ultrasound wave corresponding to the second signal distribution is different, by 180 degrees, from the transmission phase of the transmission ultrasound wave corresponding to the first signal distribution. Accordingly, by employing the reconstructing function 150g, the processing circuitry 150 generates the harmonic echo component by performing a subtracting process between the first signal distribution and the second signal distribution. It should be noted that, according to a normal pulse inversion method, a harmonic echo component is extracted by adding together signal distributions related to ultrasound wave transmissions. According to the normal pulse inversion method, the harmonic components in reception signals obtained under a first transmission condition and a second transmission condition are in mutually the same phase. However, in the present example, the transmission wave field derived from the transmission phase different by 180 degrees is correlation-processed into the reception wave field. As a result, between the first second transmission condition and the second second transmission condition, the harmonic components in the reception signals are in opposite phases rotated by 180 degrees. For this reason, to extract the harmonic component, the subtracting process is used between the first signal distribution and the second signal distribution. However, possible embodiments are not limited to the subtracting process. For example, by making the transmission phase to be the same between the first first transmission condition and the second first transmission condition and between the first second transmission condition and the second second transmission condition, i.e., by artificially generating, from the transmission conditions applied to the transmitter circuitry 9, transmission conditions that have not only mutually-different frequencies but also mutually-different phases and using the generated conditions as the first second transmission condition and the second second transmission condition, it is possible to extract the harmonic echo component by performing an adding process between the first signal distribution and the second signal distribution.

As explained above, in the second embodiment, the example was explained in which, when the plurality of ultrasound waves are transmitted, the correlation analysis is performed with respect to each of the transmission ultrasound waves, so as to extract the harmonic component on the basis of the obtained plurality of signal distributions. As a result, it is possible to carry out the harmonic imaging capable of extracting the harmonic component and to thus enhance image quality.

Modification Example of Second Embodiment

In the second embodiment, the example was explained in which, when the plurality of ultrasound waves are transmitted, the correlation analysis is performed with respect to each of the transmission ultrasound waves, so as to extract the harmonic component on the basis of the obtained plurality of signal distributions. In a modification example of the second embodiment, an example will be explained in which a correlation analysis is performed, while handling ultrasound wave transmissions in multiple sessions as one transmission condition.

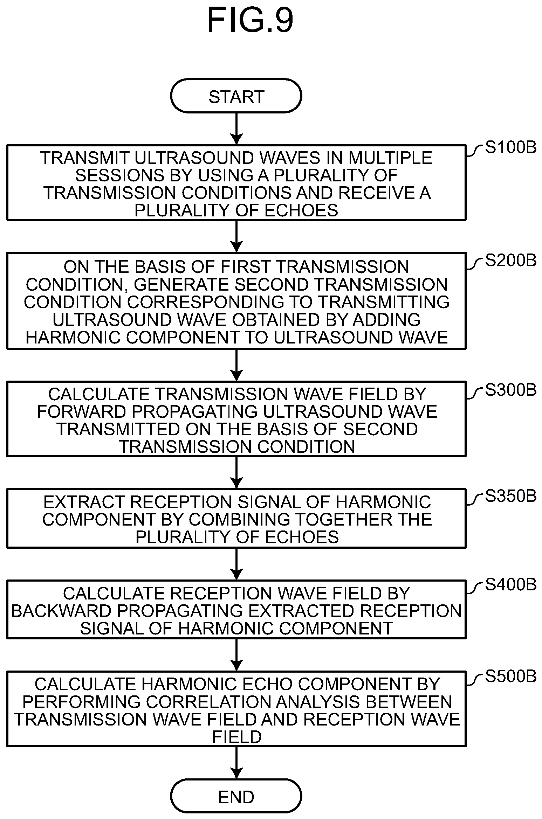

FIG. 9 is a flowchart for explaining a flow in a process performed by the ultrasound diagnosis apparatus 10 according to the modification example of the second embodiment.

To begin with, at step S100B, the transmitter circuitry 9 and the receiver circuitry 11 serving as the transmission/reception unit transmit ultrasound waves to a patient by using the first transmission condition and receive echoes from the patient. The ultrasound diagnosis apparatus 10 employs the transmitter circuitry 9 to transmit the ultrasound waves in multiple sessions that are phase-modulated into mutually the same direction by using a plurality of transmission conditions, so that the receiver circuitry 11 receives an echo from the patient with respect to each of the ultrasound waves transmitted in the multiple sessions. More specifically, the transmitter circuitry 9 transmits the ultrasound waves in the multiple sessions by using the plurality of transmission conditions, so that the receiver circuitry 11 receives the plurality of echoes corresponding to the ultrasound waves transmitted in the multiple sessions.

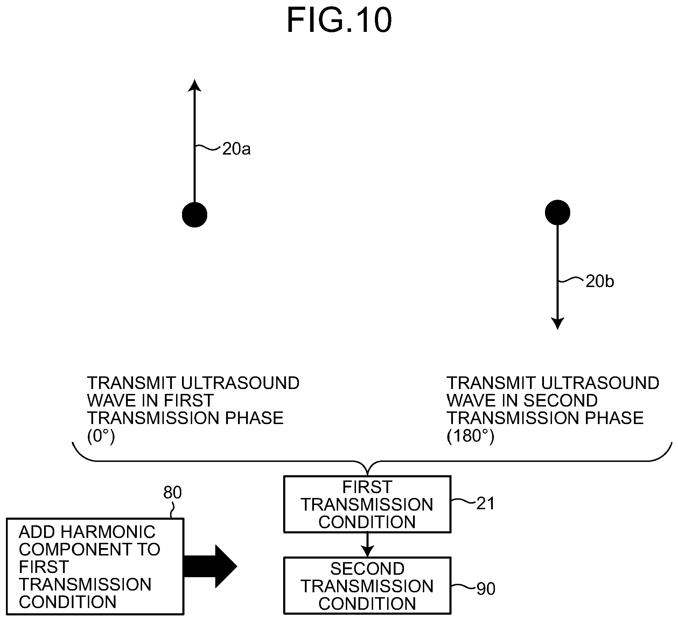

In this situation, as illustrated in FIG. 10, for example, the transmitter circuitry 9 transmits the ultrasound wave 20a at 0 degrees being the first transmission phase and transmits the ultrasound wave 20b at 180 degrees being the second transmission phase. In this situation, in the modification example of the second embodiment, the process in which the transmitter circuitry 9 transmits the ultrasound wave 20a and the ultrasound wave 20b by using the respective transmission conditions, so that the receiver circuitry 11 receives the plurality of echoes is handled as the transmission/reception unit transmitting a single ultrasound wave by using a single first transmission condition 21.

Subsequently, at step S200B, by employing the designing function 150h, the processing circuitry 150 generates a second transmission condition corresponding to transmitting an ultrasound wave obtained by adding a harmonic component to the ultrasound wave, on the basis of the first transmission condition. More specifically, by employing the designing function 150h, the processing circuitry 150 generates the second transmission condition 90 by adding the harmonic component 80 to the first transmission condition 21, while handling the process in which the transmission/reception unit transmits the ultrasound waves in the multiple sessions by using the plurality of transmission conditions and receives the plurality of echoes, as the transmission/reception unit transmitting a single ultrasound wave by using the single first transmission condition. In an example, the processing circuitry 150 may be configured to generate the second transmission condition obtained by adding, to the first transmission condition, a pulse signal in a wide band covering a propagation harmonic component or of a band itself of the propagation harmonic component

After that, at step S300B, by employing the first analyzing function 150i, the processing circuitry 150 obtains the transmission wave field 30 by forward propagating the ultrasound wave transmitted on the basis of the second transmission condition, through a forward propagation simulation. By employing the first analyzing function 150i, the processing circuitry 150 may be configured to calculate the transmission wave field 30 on the basis of the second transmission condition 90, for example, by simulating time development of a wave equation, for example, by implementing a simulation method using the FDTD method or an angular spectrum method.

Further, at step S350B, by employing the second analyzing function 150j, the processing circuitry 150 extracts a reception signal containing a harmonic component, by combining together the plurality of echoes received at step S100B. In an example, when the transmitter circuitry 9 performs ultrasound wave transmissions, by regarding ultrasound wave transmissions in two sessions having transmission phases that are different from each other by 180 degrees as one set, the processing circuitry 150 may be configured, by employing the second analyzing function 150j, to extract a reception signal containing a second-order harmonic component, by simply adding together the plurality of echoes received at step S100B. More specifically, at step S100B, a reception signal of transducer element containing a second-order harmonic component is generated by adding the reception signals of transducer elements obtained by the ultrasound wave transmissions in two sessions having different transmission phases. In another example, when the transmitter circuitry 9 performs ultrasound wave transmissions, by regarding ultrasound wave transmissions in three sessions having transmission phases that are different from one another by 120 degrees as one set, the processing circuitry 150 may be configured, by employing the second analyzing function 150j, to extract a reception signal containing a third-order harmonic component, by simply adding together the plurality of echoes received at step S100B.

Subsequently, at step S400B, by employing the second analyzing function 150j, the processing circuitry 150 calculates the reception wave field 60 by backward propagating the reception signal 50 based on the echoes acquired at step S100. In other words, by employing the second analyzing function 150j, the processing circuitry 150 obtains the reception wave field 60, by backward propagating the reception signal 50 extracted at step S350B on the basis of the echoes acquired at step S100. For example, by employing the second analyzing function 150j, the processing circuitry 150 may be configured to obtain the reception wave field 60 by backward propagating the echoes corresponding to the transmission condition, for example, by simulating time development of a wave equation while implementing the FDTD method or implementing a simulation method using an angular spectrum method.

After that, at step S500B, by employing the reconstructing function 150g, the processing circuitry 150 generates a harmonic echo component by performing a correlation analysis between the transmission wave field 30 calculated at step S300B and the reception wave field 60 calculated at step S400B. In an example, by employing the reconstructing function 150g, the processing circuitry 150 may be configured to perform the correlation analysis by evaluating the right-hand side of Expression (1) between the transmission wave field 30 and the reception wave field 60 and to obtain a resulting image I. Because the resulting image I contains the harmonic echo component, the processing circuitry 150 is able to extract the harmonic component by performing the correlation analysis, while employing the reconstructing function 150g.

As explained above, in the modification example of the second embodiment, when the plurality of ultrasound waves are transmitted, the ultrasound waves in the multiple sessions are combined together at first, before performing the correlation analysis, so as to extract the harmonic component. With this arrangement, it is possible to carry out the harmonic imaging capable of extracting the harmonic component and to thus enhance image quality.

Third Embodiment

Possible embodiments are not limited to the above examples. In the first embodiment, the example was explained in which, when the forward propagation or backward propagation simulation is performed on the transmission wave fields and the reception wave fields at step S300 and step S400, the simulation is performed without taking non-linear effects of a medium into consideration. However, possible embodiments are not limited to this example. In a third embodiment, an example will be explained in which a simulation is performed while considering the non-linear effects of the medium.

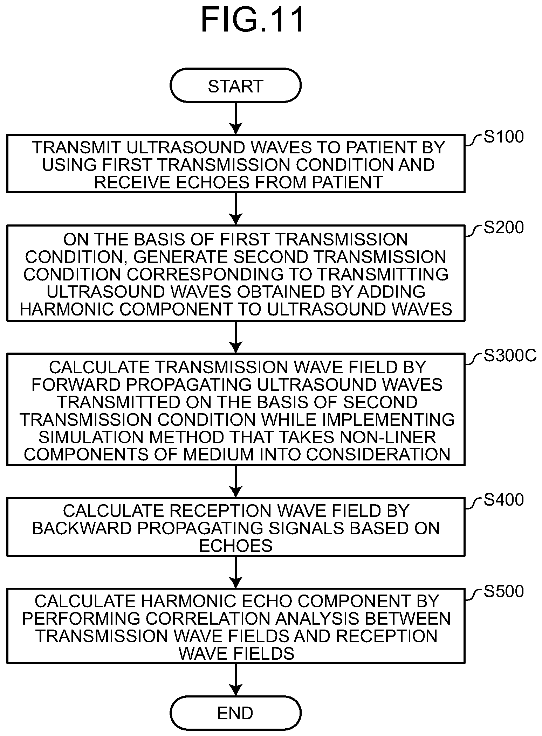

FIG. 11 is a flowchart for explaining a flow in a process performed by the ultrasound diagnosis apparatus 10 according to the third embodiment. In this situation, because the processes other than step S300C are the same as those explained in the first embodiment with reference to FIG. 5, explanations of those processes will not be repeated.

At step S300C, by employing the first analyzing function 150i, the processing circuitry 150 calculates the transmission wave fields 30 by forward propagating the ultrasound waves transmitted on the basis of the second transmission condition, by implementing a simulation method that takes non-linear components of the medium into consideration. In an example, by employing the first analyzing function 150i, the processing circuitry 150 may be configured to calculate the transmission wave fields 30 by forward propagating the ultrasound waves transmitted on the basis of the second transmission condition, while implementing a simulation method using a finite element method, for example. By performing the simulation taking the non-linear effects of the medium into consideration, it is expected that image quality will be further enhanced.

Similarly, at step S400, by employing the second analyzing function 150j, the processing circuitry 150 may obtain the reception wave fields 60 by backward propagating the signals based on the echoes, while implementing a simulation method that takes the non-linear components of the medium into consideration.

Further, by employing the designing function 150h, the processing circuitry 150 may be configured to measure, in advance, a non-linear parameter of the medium before performing the process at step S100 and to store the measured non-linear parameter of the medium into the memory 132. After that, at step S300C or step S400, the processing circuitry 150 may be configured to invoke the stored non-linear parameter of the medium from the memory 132 so as to use the parameter for the simulation.

In yet another example, by employing the receiving function 150e, the processing circuitry 150 may be configured to receive, from the user, a value of the non-linear parameter of the medium via a user interface and to store the received non-linear parameter of the medium into the memory 132. After that, subsequently, at step S300C or step S400, the processing circuitry 150 may be configured to invoke the stored non-linear parameter of the medium from the memory 132 and to use the parameter for the simulation.

As explained above, the ultrasound diagnosis apparatus 10 according to the third embodiment is able to realize further enhancement of image quality, by performing the simulation that takes the non-linear parameter of the medium into consideration.

According to at least one aspect of the embodiments described above, it is possible to enhance image quality.

While certain embodiments have been described, these embodiments have been presented by way of example only, and are not intended to limit the scope of the inventions.

**Claims**

1. An ultrasound diagnosis apparatus comprising:

   transmitter/receiver circuitry (9, 11) configured to transmit an ultrasound wave to an examined subject by using a first transmission condition and to receive an echo from the examined subject; and
   processing circuitry (150) configured

   to generate, on a basis of the first transmission condition, a second transmission condition corresponding to transmitting an ultrasound wave obtained by adding a harmonic component to the ultrasound wave,
   to obtain a transmission wave field by forward propagating the ultrasound wave transmitted on a basis of the second transmission condition,
   to obtain a reception wave field by backward propagating a signal based on the echo, and
   to generate a harmonic echo component by performing a correlation analysis between the transmission wave field and the reception wave field.

2. The ultrasound diagnosis apparatus according to claim 1, wherein, as the second transmission condition, the processing circuitry (150) is configured to generate a transmission condition that has a frequency band of a harmonic component in contrast to the first transmission condition and that is same as the first transmission condition except for the frequency band.

3. The ultrasound diagnosis apparatus according to claim 1 or 2, wherein the transmitter/receiver circuitry (9, 11) is configured to transmit ultrasound waves in multiple sessions that are phase-modulated into a mutually same direction and to receive the echo from the examined subject with respect to each of the ultrasound waves transmitted in the multiple sessions.

4. The ultrasound diagnosis apparatus according to any one of claims 1 to 3, wherein

   the transmiter/receiver circuitry is configured to transmit ultrasound waves in multiple sessions by using a plurality of first transmission conditions including the first transmission condition and to receive a plurality of echoes including the echo,
   the processing circuitry (150) is configured to generate a plurality of second transmission conditions including the second transmission condition and respectively corresponding to the plurality of first transmission conditions,
   the processing circuitry (150) is configured to obtain the transmission wave field with respect to each of the plurality of second transmission conditions,
   the processing circuitry (150) is configured to obtain the reception wave field with respect to each of the echoes respectively corresponding to the plurality of first transmission conditions, and
   the processing circuitry (150) is configured to perform the correlation analysis between the transmission wave fields and the reception wave fields with respect to the second transmission conditions respectively corresponding to the first transmission conditions and to generate the harmonic echo component on a basis of a result of the correlation analysis.

5. The ultrasound diagnosis apparatus according to claim 4, wherein

   the transmitter/receiver circuitry (9, 11) is configured to transmit the ultrasound wave by using a first one of the first transmission condition in a first transmission phase to receive a first echo and configured to transmit the ultrasound wave by using a second one of the first transmission condition in a second transmission phase different from the first transmission phase by 180 degrees to receive a second echo,

the processing circuitry (150) is configured to generate a first one of the second transmission condition on a basis of the first one of the first transmission condition and to generate a second one of the second transmission condition on a basis of the second one of the first transmission condition,

the processing circuitry (150) is configured to obtain a first transmission wave field on a basis of the first one of the second transmission condition,

the processing circuitry (150) is configured to obtain a second transmission wave field on a basis of the second one of the second transmission condition,

the processing circuitry (150) is configured to obtain a first reception wave field on a basis of the first echo and to obtain a second reception wave field on a basis of the second echo, and

the processing circuitry (150) is configured to generate a first signal distribution by performing a correlation analysis between the first transmission wave field and the first reception wave field, to generate a second signal distribution by performing a correlation analysis between the second transmission wave field and the second reception wave field, and to generate the harmonic echo component on a basis of the first signal distribution and the second signal distribution.

6. The ultrasound diagnosis apparatus according to claim 5, wherein the processing circuitry (150) is configured to generate the harmonic echo component by performing a subtracting process between the first signal distribution and the second signal distribution.

7. The ultrasound diagnosis apparatus according to claim 1, wherein

the processing circuitry (150) is configured to transmit ultrasound waves in multiple sessions by using a plurality of transmission conditions and to receive a plurality of echoes including the echo,

the processing circuitry (150) is configured to handle a process in which the transmitter/receiver circuitry (9, 11) transmits the ultrasound waves in the multiple sessions by using the plurality of transmission conditions and receives the plurality of echoes, as the transmitter/receiver circuitry (9, 11) transmitting a single ultrasound wave by using the first transmission condition being singular,

the processing circuitry (150) is configured to generate the second transmission condition on a basis of the first transmission condition,

the processing circuitry (150) is configured to obtain the transmission wave field on a basis of the second transmission condition,

the processing circuitry (150) is configured to obtain the reception wave field on a basis of the plurality of echoes, and

the processing circuitry (150) is configured to generate the harmonic echo component by performing the correlation analysis between the transmission wave field and the reception wave field.

8. The ultrasound diagnosis apparatus according to claim 7, wherein

the processing circuitry (150) is configured to extract a reception signal containing a harmonic component by combining the plurality of echoes together, and

the processing circuitry (150) is configured to obtain the reception wave field on a basis of the extracted reception signal.

9. The ultrasound diagnosis apparatus according to any one of claims 1 to 8, wherein the processing circuitry (150) is configured to obtain the transmission wave field by forward propagating the ultrasound wave while implementing a simulation method that takes a non-linear component of a medium into consideration.

10. The ultrasound diagnosis apparatus according to any one of claims 1 to 8, wherein the processing circuitry (150) is configured to obtain the reception wave field by backward propagating the signal based on the echo while implementing a simulation method that takes a non-linear component of a medium into consideration.

11. The ultrasound diagnosis apparatus according to any oee of claims 1 to 10, wherein the processing circuitry (150) is configured to implement a simulation method that uses an angular spectrum method.

12. An ultrasound diagnosis method comprising:

transmitting an ultrasound wave to an examined subject by using a first transmission condition and receiving an echo from the examined subject;

generating, on a basis of the first transmission condition, a second transmission condition corresponding to transmitting an ultrasound wave obtained by adding a harmonic component to the ultrasound wave;
obtaining a transmission wave field by forward propagating the ultrasound wave transmitted on a basis of the second transmission condition;
obtaining a reception wave field by backward propagating a signal based on the echo; and
generating a harmonic echo component by performing a correlation analysis between the transmission wave field and the reception wave field.

**Patentansprüche**

1. Ultraschalldiagnoseeinrichtung umfassend:

   Sende-/Empfangsschaltlogik (9, 11), die so konfiguriert ist, dass sie unter Verwendung einer ersten Sendebedingung eine Ultraschallwelle zu einem untersuchten Probanden sendet und ein Echo von dem untersuchten Probanden empfängt; und
   Verarbeitungsschaltlogik (150), konfiguriert zum

   Erzeugen, auf der Grundlage der ersten Sendebedingung, einer zweiten Sendebedingung, die dem Senden einer Ultraschallwelle entspricht, die durch Hinzufügen einer harmonischen Komponente zu der Ultraschallwelle erhalten wurde,
   Erhalten eines Sendewellenfelds durch Vorwärtsausbreitung der auf der Grundlage der zweiten Sendebedingung gesendeten Ultraschallwelle,
   Erhalten eines Empfangswellenfelds durch Rückwärtsausbreitung eines auf dem Echo basierendes Signals, und
   Erzeugen einer harmonische Echokomponente, indem eine Korrelationsanalyse zwischen dem Sendewellenfeld und dem Empfangswellenfeld durchgeführt wird.

2. Ultraschalldiagnoseeinrichtung nach Anspruch 1, wobei die Verarbeitungsschaltlogik (150), als die zweite Sendebedingung, so konfiguriert ist, dass sie eine Sendebedingung erzeugt, die ein Frequenzband einer harmonischen Komponente im Gegensatz zu der ersten Sendebedingung aufweist und mit der ersten Sendebedingung bis auf das Frequenzband übereinstimmt.

3. Ultraschalldiagnoseeinrichtung nach Anspruch 1 oder 2, wobei die Sende-/Empfangsschaltlogik (9, 11) so konfiguriert ist, dass sie Ultraschallwellen in mehreren Sitzungen sendet, die in eine gegenseitig gleiche Richtung phasenmoduliert sind, und dass sie das Echo des untersuchten Objekts in Bezug auf jede der in den mehreren Sitzungen gesendeten Ultraschallwellen empfängt.

4. Ultraschalldiagnoseeinrichtung nach einem der Ansprüche 1 bis 3, wobei

   die Sende-/Empfangsschaltlogik so konfiguriert ist, dass sie Ultraschallwellen in mehreren Sitzungen unter Verwendung einer Vielzahl von ersten Sendebedingungen, einschließlich der ersten Sendebedingung, sendet und eine Vielzahl von Echos, einschließlich des Echos, empfängt,
   die Verarbeitungsschaltlogik (150) so konfiguriert ist, dass sie eine Vielzahl von zweiten Sendebedingungen erzeugt, die die zweite Sendebedingung einschließen und jeweils der Vielzahl von ersten Sendebedingungen entsprechen,
   die Verarbeitungsschaltlogik (150) so konfiguriert ist, dass sie das Sendewellenfeld in Bezug auf jede der Vielzahl von zweiten Sendebedingungen erhält,
   die Verarbeitungsschaltlogik (150) so konfiguriert ist, dass sie das Empfangswellenfeld in Bezug auf jedes der Echos, die jeweils der Vielzahl von ersten Sendebedingungen entsprechen, erhält, und
   die Verarbeitungsschaltlogik (150) so konfiguriert ist, dass sie die Korrelationsanalyse zwischen den Sendewellenfeldern und den Empfangswellenfeldern in Bezug auf die zweiten Sendebedingungen, die jeweils den ersten Sendebedingungen entsprechen, durchführt und die harmonische Echokomponente auf der Grundlage eines Ergebnisses der Korrelationsanalyse erzeugt.

5. Ultraschalldiagnoseeinrichtung nach Anspruch 4, wobei

   die Sende-/Empfangsschaltlogik (9, 11) so konfiguriert ist, dass sie Ultraschallwelle unter Verwendung einer ersten von der ersten Sendebedingung in einer ersten Sendephase sendet, um ein erstes Echo zu empfangen, und so konfiguriert ist, dass sie die Ultraschallwelle unter Verwendung einer zweiten von der ersten Sendebedingung in einer zweiten Sendephase sendet, die sich von der ersten Sendephase um 180 Grad unterscheidet, um ein zweites Echo zu empfangen,

die Verarbeitungsschaltlogik (150) so konfiguriert ist, dass sie eine erste von der zweiten Sendebedingung auf der Grundlage der ersten von der ersten Sendebedingung erzeugt und eine zweite von der zweiten Sendebedingung auf der Grundlage der zweiten von der ersten Sendebedingung erzeugt,

die Verarbeitungsschaltlogik (150) so konfiguriert ist, dass sie ein erstes Sendewellenfeld auf der Grundlage der ersten von der zweiten Sendebedingung erhält,

die Verarbeitungsschaltlogik (150) so konfiguriert ist, dass sie ein zweites Sendewellenfeld auf der Grundlage der zweiten von der zweiten Sendebedingung erhält,

die Verarbeitungsschaltlogik (150) so konfiguriert ist, dass sie ein erstes Empfangswellenfeld auf der Grundlage des ersten Echos erhält und ein zweites Empfangswellenfeld auf der Grundlage des zweiten Echos erhält,

die Verarbeitungsschaltlogik (150) konfiguriert ist, um eine erste Signalverteilung zu erzeugen, indem sie eine Korrelationsanalyse zwischen dem ersten Sendewellenfeld und dem ersten Empfangswellenfeld durchführt, eine zweite Signalverteilung zu erzeugen, indem sie eine Korrelationsanalyse zwischen dem zweiten Sendewellenfeld und dem zweiten Empfangswellenfeld durchführt, und die harmonische Echokomponente auf der Grundlage der ersten Signalverteilung und der zweiten Signalverteilung zu erzeugen.

6. Ultraschalldiagnoseeinrichtung nach Anspruch 5, wobei die Verarbeitungsschaltlogik (150) so konfiguriert ist, dass sie die harmonische Echokomponente erzeugt, indem sie einen Subtraktionsprozess zwischen der ersten Signalverteilung und der zweiten Signalverteilung durchführt.

7. Ultraschalldiagnoseeinrichtung nach Anspruch 1, wobei

die Verarbeitungsschaltlogik (150) so konfiguriert ist, dass sie Ultraschallwellen **in** mehreren Sitzungen unter Verwendung einer Vielzahl von Sendebedingungen sendet und dass sie eine Vielzahl von Echos, einschließlich des Echos, empfängt,

die Verarbeitungsschaltlogik (150) so konfiguriert ist, dass sie einen Prozess handhabt, bei dem die Sende-/Empfangsschaltlogik (9, 11) die Ultraschallwellen in den mehreren Sitzungen unter Verwendung der Vielzahl von Sendebedingungen sendet und die Vielzahl von Echos empfängt, während die Sende-/Empfangsschaltlogik (9, 11) eine einzelne Ultraschallwelle unter Verwendung der ersten Sendebedingung sendet, die singulär ist,

die Verarbeitungsschaltlogik (150) so konfiguriert ist, dass sie die zweite Sendebedingung auf der Grundlage der ersten Sendebedingung erzeugt,

die Verarbeitungsschaltlogik (150) so konfiguriert ist, dass sie das Sendewellenfeld auf der Grundlage der zweiten Sendebedingung erhält,

die Verarbeitungsschaltlogik (150) so konfiguriert ist, dass sie das Empfangswellenfeld auf der Grundlage der Vielzahl von Echos erhält, und

die Verarbeitungsschaltlogik (150) so konfiguriert ist, dass sie die harmonische Echokomponente erzeugt, indem sie die Korrelationsanalyse zwischen dem Sendewellenfeld und dem Empfangswellenfeld durchführt.

8. Ultraschalldiagnoseeinrichtung nach Anspruch 7, wobei

die Verarbeitungsschaltlogik (150) so konfiguriert ist, dass sie ein Empfangssignal extrahiert, das eine harmonische Komponente enthält, indem sie die Vielzahl von Echos miteinander kombiniert, und

die Verarbeitungsschaltlogik (150) so konfiguriert ist, dass das Empfangswellenfeld auf der Grundlage des extrahierten Empfangssignals erhält.

9. Ultraschalldiagnoseeinrichtung nach einem der Ansprüche 1 bis 8, wobei die Verarbeitungsschaltlogik (150) so konfiguriert ist, dass sie das Sendewellenfeld durch Vorwärtsausbreitung der Ultraschallwelle erhält, während sie ein Simulationsverfahren implementiert, das eine nichtlineare Komponente eines Mediums berücksichtigt.

10. Ultraschalldiagnoseeinrichtung nach einem der Ansprüche 1 bis 8, wobei die Verarbeitungsschaltlogik (150) so konfiguriert ist, dass sie das Empfangswellenfeld durch Rückwärtsausbreitung des Signals auf der Grundlage des Echos erhält, während sie ein Simulationsverfahren implementiert, das eine nichtlineare Komponente eines Mediums berücksichtigt.

11. Ultraschalldiagnoseeinrichtung nach einem der Ansprüche 1 bis 10, wobei die Verarbeitungsschaltlogik (150) so konfiguriert ist, dass ein Simulationsverfahren implementiert, das ein Winkelspektrumverfahren verwendet.

12. Ultraschalldiagnoseverfahren, umfassend:

Senden einer Ultraschallwelle zu einem untersuchten Probanden unter Verwendung einer ersten Sendebedingung und Empfangen eines Echos von dem untersuchten Probanden;
Erzeugen, auf der Grundlage der ersten Sendebedingung, einer zweiten Sendebedingung, die dem Senden einer Ultraschallwelle entspricht, die durch Hinzufügen einer harmonischen Komponente zu der Ultraschallwelle erhalten wird;
Erhalten eines Sendewellenfelds durch Vorwärtsausbreitung der auf der Grundlage der zweiten Sendebedingung sendeten Ultraschallwelle;
Erhalten eines Empfangswellenfeld durch Rückwärtsausbreitung eines auf dem Echo basierenden Signals; und
Erzeugen einer harmonische Echokomponente, indem eine Korrelationsanalyse zwischen dem Sendewellenfeld und dem Empfangswellenfeld durchgeführt wird.

## Revendications

1. Appareil de diagnostic par ultrasons comprenant :

un circuit émetteur/récepteur (9, 11) configuré pour transmettre une onde ultrasonore à un sujet examiné en utilisant une première condition de transmission et pour recevoir un écho du sujet examiné ; et
un circuit de traitement (150) configuré

pour générer, sur la base de la première condition de transmission, une deuxième condition de transmission correspondant à la transmission d'une onde ultrasonore obtenue en ajoutant une composante harmonique à l'onde ultrasonore,
pour obtenir un champ d'ondes de transmission en propageant vers l'avant l'onde ultrasonore transmise sur la base de la deuxième condition de transmission,
pour obtenir un champ d'ondes de réception en propageant dans le sens inverse un signal basé sur l'écho, et
pour générer une composante d'écho harmonique en effectuant une analyse de corrélation entre le champ d'ondes de transmission et le champ d'ondes de réception.

2. Appareil de diagnostic par ultrasons selon la revendication 1, dans lequel, comme deuxième condition de transmission, le circuit de traitement (150) est configuré pour générer une condition de transmission qui présente une bande de fréquence d'une composante harmonique par rapport à la première condition de transmission et qui est identique à la première condition de transmission à l'exception de la bande de fréquence.

3. Appareil de diagnostic par ultrasons selon la revendication 1 ou 2, dans lequel le circuit émetteur/récepteur (9, 11) est configuré pour transmettre des ondes ultrasonores en plusieurs sessions qui sont modulées en phase dans une direction mutuellement identique et pour recevoir l'écho du sujet examiné par rapport à chacune des ondes ultrasonores transmises dans les multiples sessions.

4. Appareil de diagnostic par ultrasons selon l'une quelconque des revendications 1 à 3, dans lequel

le circuit émetteur/récepteur est configuré pour transmettre des ondes ultrasonores en plusieurs sessions en utilisant une pluralité de premières conditions de transmission, y compris la première condition de transmission, et pour recevoir une pluralité d'échos, y compris l'écho,
le circuit de traitement (150) est configuré pour générer une pluralité de deuxièmes conditions de transmission, y compris la deuxième condition de transmission et correspondant respectivement à la pluralité de premières conditions de transmission,
le circuit de traitement (150) est configuré pour obtenir le champ d'onde de transmission par rapport à chacune de la pluralité de deuxièmes conditions de transmission,
le circuit de traitement (150) est configuré pour obtenir le champ d'onde de réception par rapport à chacun des échos correspondant respectivement à la pluralité des premières conditions de transmission, et
le circuit de traitement (150) est configuré pour effectuer l'analyse de corrélation entre les champs d'ondes de transmission et les champs d'ondes de réception par rapport aux deuxièmes conditions de transmission correspondant respectivement aux premières conditions de transmission et pour générer la composante d'écho harmonique sur la base d'un résultat de l'analyse de corrélation.

5. Appareil de diagnostic par ultrasons selon la revendication 4, dans lequel

le circuit émetteur/récepteur (9, 11) est configuré pour transmettre l'onde ultrasonore en utilisant une première condition de transmission dans une première phase de transmission pour recevoir un premier écho et configuré pour transmettre l'onde ultrasonore en utilisant une deuxième de la première condition de transmission dans une deuxième phase de transmission différente de la première phase de transmission de 180 degrés pour recevoir un deuxième écho.

le circuit de traitement (150) est configuré pour générer une première de la deuxième condition de transmission sur la base de la première de la première condition de première transmission et pour générer une deuxième de la deuxième condition de transmission sur la base de la deuxième de la première condition de transmission,

le circuit de traitement (150) est configuré pour obtenir un premier champ d'ondes de transmission sur la base de la première de la deuxième condition de transmission,

le circuit de traitement (150) est configuré pour obtenir un deuxième champ d'ondes de transmission sur la base de la deuxième de la deuxième condition de transmission,

le circuit de traitement (150) est configuré pour obtenir un premier champ d'ondes de réception à partir du premier écho et pour obtenir un deuxième champ d'ondes de réception sur la base du deuxième écho, et

le circuit de traitement (150) est configuré pour générer une première distribution de signal en effectuant une analyse de corrélation entre le premier champ d'ondes de transmission et le premier champ d'ondes de réception pour générer une deuxième distribution de signal en effectuant une analyse de corrélation entre le deuxième champ d'ondes de transmission et le deuxième champ d'ondes de réception et pour générer la composante d'écho harmonique sur la base de la première distribution de signal et de la deuxième distribution de signal.

6. Appareil de diagnostic par ultrasons selon la revendication 5, dans lequel le circuit de traitement (150) est configuré pour générer la composante d'écho harmonique en effectuant un processus de soustraction entre la première distribution de signal et la deuxième distribution de signal.

7. Appareil de diagnostic par ultrasons selon la revendication 1, dans lequel

le circuit de traitement (150) est configuré pour transmettre des ondes ultrasonores en plusieurs sessions en utilisant une pluralité de conditions de transmission et pour recevoir une pluralité d'échos, y compris l'écho,

le circuit de traitement (150) est configuré pour gérer un processus dans lequel le circuit émetteur/récepteur (9, 11) transmet les ondes ultrasonores lors des plusieurs sessions en utilisant plusieurs conditions de transmission et reçoit la pluralité d'échos, tandis que le circuit émetteur/récepteur (9, 11) transmet une seule onde ultrasonore en utilisant la première condition de transmission qui est unique,

le circuit de traitement (150) est configuré pour générer la deuxième condition de transmission sur la base de la première condition de transmission,

le circuit de traitement (150) est configuré pour obtenir le champ d'ondes de transmission sur la base de la deuxième condition de transmission,

le circuit de traitement (150) est configuré pour obtenir le champ d'ondes de réception sur la base de la pluralité d'échos, et

le circuit de traitement (150) est configuré pour générer la composante d'écho harmonique en effectuant l'analyse de corrélation entre le champ d'ondes de transmission et le champ d'ondes de réception.

8. Appareil de diagnostic par ultrasons selon la revendication 7, dans lequel

le circuit de traitement (150) est configuré pour extraire un signal de réception contenant une composante harmonique en combinant la pluralité d'échos, et

le circuit de traitement (150) est configuré pour obtenir le champ d'ondes de réception sur la base du signal de réception extrait.

9. Appareil de diagnostic par ultrasons selon l'une quelconque des revendications 1 à 8, dans lequel le circuit de traitement (150) est configuré pour obtenir le champ d'ondes de transmission en propageant l'onde ultrasonore vers l'avant tout en mettant en œuvre un procédé de simulation qui prend en considération une composante non linéaire d'un milieu.

10. Appareil de diagnostic par ultrasons selon l'une quelconque des revendications 1 à 8, dans lequel le circuit de traitement (150) est configuré pour obtenir le champ d'ondes de réception en propageant le signal basé sur l'écho dans le sens inverse tout en mettant en œuvre un procédé de simulation qui prend en considération une composante non linéaire d'un milieu.

**11.** Appareil de diagnostic par ultrasons selon l'une quelconque des revendications 1 à 10, dans lequel le circuit de traitement (150) est configuré pour mettre en œuvre un procédé de simulation qui utilise un procédé de spectre angulaire.

**12.** Procédé de diagnostic par ultrasons comprenant : la transmission d'une onde ultrasonore à un sujet examiné en utilisant une première condition de transmission et la réception d'un écho du sujet examiné ;

la génération, sur la base de la première condition de transmission, d'une deuxième condition de transmission correspondant à la transmission d'une onde ultrasonore obtenue en ajoutant une composante harmonique à l'onde ultrasonore ;
l'obtention d'un champ d'ondes de transmission en propageant vers l'avant l'onde ultrasonore transmise sur la base de la deuxième condition de transmission ;
l'obtention d'un champ d'ondes de réception en propageant dans le sens inverse un signal basé sur l'écho ; et
la génération d'une composante d'écho harmonique en effectuant une analyse de corrélation entre le champ d'ondes de transmission et le champ d'ondes de réception.

# FIG.1

**ULTRASOUND DIAGNOSIS APPARATUS** ⌇10

**MEDICAL IMAGE PROCESSING APPARATUS** ⌇100

ULTRASOUND PROBE ⌇5

TRANSMITTER CIRCUITRY ⌇9

RECEIVER CIRCUITRY ⌇11

MEMORY ⌇132

INPUT APPARATUS ⌇134

DISPLAY ⌇135

PROCESSING CIRCUITRY ⌇150

B-MODE PROCESSING FUNCTION ⌇150a

DOPPLER PROCESSING FUNCTION ⌇150b

GENERATING FUNCTION ⌇150c

DISPLAY CONTROLLING FUNCTION ⌇150d

RECEIVING FUNCTION ⌇150e

CONTROLL-ING FUNCTION ⌇150f

RE-CONSTRUCT-ING FUNCTION ⌇150g

DESIGNING FUNCTION ⌇150h

FIRST ANALYZING FUNCTION ⌇150i

SECOND ANALYZING FUNCTION ⌇150j

# FIG.2

# FIG.3

START

TRANSMIT ULTRASOUND WAVES TO PATIENT BY USING FIRST TRANSMISSION CONDITION AND RECEIVE ECHOES FROM PATIENT ⌐S100

CALCULATE TRANSMISSION WAVE FIELDS BY FORWARD PROPAGATING ULTRASOUND WAVES TRANSMITTED ON THE BASIS OF FIRST TRANSMISSION CONDITION ⌐S300X

CALCULATE RECEPTION WAVE FIELDS BY BACKWARD PROPAGATING SIGNALS BASED ON ECHOES ⌐S400

GENERATE IMAGE BY PERFORMING CORRELATION ANALYSIS BETWEEN TRANSMISSION WAVE FIELDS AND RECEPTION WAVE FIELDS ⌐S500

END

# FIG.4

EP 4 517 375 B1

# FIG.5

START

TRANSMIT ULTRASOUND WAVES TO PATIENT BY USING FIRST TRANSMISSION CONDITION AND RECEIVE ECHOES FROM PATIENT — S100

ON THE BASIS OF FIRST TRANSMISSION CONDITION, GENERATE SECOND TRANSMISSION CONDITION CORRESPONDING TO TRANSMITTING ULTRASOUND WAVES OBTAINED BY ADDING HARMONIC COMPONENT TO ULTRASOUND WAVES — S200

CALCULATE TRANSMISSION WAVE FIELDS BY FORWARD PROPAGATING ULTRASOUND WAVES TRANSMITTED ON THE BASIS OF SECOND TRANSMISSION CONDITION — S300

CALCULATE RECEPTION WAVE FIELDS BY BACKWARD PROPAGATING SIGNALS BASED ON ECHOES — S400

CALCULATE HARMONIC ECHO COMPONENT BY PERFORMING CORRELATION ANALYSIS BETWEEN TRANSMISSION WAVE FIELDS AND RECEPTION WAVE FIELDS — S500

END

# FIG.6

START

TRANSMIT ULTRASOUND WAVE BY USING FIRST FIRST TRANSMISSION CONDITION IN FIRST TRANSMISSION PHASE TO RECEIVE FIRST ECHO AND TRANSMIT ULTRASOUND WAVE BY USING SECOND FIRST TRANSMISSION CONDITION IN SECOND TRANSMISSION PHASE DIFFERENT FROM FIRST TRANSMISSION PHASE BY 180 DEGREES TO RECEIVE SECOND ECHO —S100A

GENERATE FIRST SECOND TRANSMISSION CONDITION ON THE BASIS OF FIRST FIRST TRANSMISSION CONDITION AND GENERATE SECOND SECOND TRANSMISSION CONDITION ON THE BASIS OF SECOND FIRST TRANSMISSION CONDITION —S200A

OBTAIN FIRST TRANSMISSION WAVE FIELD BY FORWARD PROPAGATING ULTRASOUND WAVE TRANSMITTED ON THE BASIS OF FIRST SECOND TRANSMISSION CONDITION AND CALCULATE SECOND TRANSMISSION WAVE FIELD BY FORWARD PROPAGATING ULTRASOUND WAVE TRANSMITTED ON THE BASIS OF SECOND SECOND TRANSMISSION CONDITION —S300A

CALCULATE FIRST RECEPTION WAVE FIELD BY BACKWARD PROPAGATING SIGNAL BASED ON FIRST ECHO AND CALCULATE SECOND RECEPTION WAVE FIELD ON THE BASIS OF SECOND ECHO —S400A

GENERATE FIRST SIGNAL DISTRIBUTION BY PERFORMING CORRELATION ANALYSIS BETWEEN FIRST TRANSMISSION WAVE FIELD AND FIRST RECEPTION WAVE FIELD AND GENERATE SECOND SIGNAL DISTRIBUTION BY PERFORMING CORRELATION ANALYSIS BETWEEN SECOND TRANSMISSION WAVE FIELD AND SECOND RECEPTION WAVE FIELD —S500A

GENERATE HARMONIC ECHO COMPONENT BY PERFORMING SUBTRACTING PROCESS BETWEEN FIRST SIGNAL DISTRIBUTION AND SECOND SIGNAL DISTRIBUTION —S550A

END

# FIG.7

EP 4 517 375 B1

# FIG.8

20a

20b

20c

21a

TRANSMIT ULTRASOUND
WAVE IN FIRST
TRANSMISSION PHASE
(0°)
(FIRST FIRST
TRANSMISSION
CONDITION)

21b

TRANSMIT ULTRASOUND
WAVE IN SECOND
TRANSMISSION PHASE
(120°)
(SECOND FIRST
TRANSMISSION
CONDITION)

21c

TRANSMIT ULTRASOUND
WAVE IN THIRD
TRANSMISSION PHASE
(240°)
(THIRD FIRST
TRANSMISSION
CONDITION)

80

ADD HARMONIC
COMPONENT TO
FIRST
TRANSMISSION
CONDITION

90a

FIRST SECOND
TRANSMISSION
CONDITION

90b

SECOND SECOND
TRANSMISSION
CONDITION

90c

THIRD SECOND
TRANSMISSION
CONDITION

# FIG.9

START

TRANSMIT ULTRASOUND WAVES IN MULTIPLE SESSIONS BY USING A PLURALITY OF TRANSMISSION CONDITIONS AND RECEIVE A PLURALITY OF ECHOES — S100B

ON THE BASIS OF FIRST TRANSMISSION CONDITION, GENERATE SECOND TRANSMISSION CONDITION CORRESPONDING TO TRANSMITTING ULTRASOUND WAVE OBTAINED BY ADDING HARMONIC COMPONENT TO ULTRASOUND WAVE — S200B

CALCULATE TRANSMISSION WAVE FIELD BY FORWARD PROPAGATING ULTRASOUND WAVE TRANSMITTED ON THE BASIS OF SECOND TRANSMISSION CONDITION — S300B

EXTRACT RECEPTION SIGNAL OF HARMONIC COMPONENT BY COMBINING TOGETHER THE PLURALITY OF ECHOES — S350B

CALCULATE RECEPTION WAVE FIELD BY BACKWARD PROPAGATING EXTRACTED RECEPTION SIGNAL OF HARMONIC COMPONENT — S400B

CALCULATE HARMONIC ECHO COMPONENT BY PERFORMING CORRELATION ANALYSIS BETWEEN TRANSMISSION WAVE FIELD AND RECEPTION WAVE FIELD — S500B

END

# FIG.10

20a

20b

TRANSMIT ULTRASOUND WAVE IN FIRST TRANSMISSION PHASE (0°)

TRANSMIT ULTRASOUND WAVE IN SECOND TRANSMISSION PHASE (180°)

80

ADD HARMONIC COMPONENT TO FIRST TRANSMISSION CONDITION

FIRST TRANSMISSION CONDITION  ~21

SECOND TRANSMISSION CONDITION  ~90

# FIG.11

START

TRANSMIT ULTRASOUND WAVES TO PATIENT BY USING FIRST TRANSMISSION CONDITION AND RECEIVE ECHOES FROM PATIENT ⌐S100

ON THE BASIS OF FIRST TRANSMISSION CONDITION, GENERATE SECOND TRANSMISSION CONDITION CORRESPONDING TO TRANSMITTING ULTRASOUND WAVES OBTAINED BY ADDING HARMONIC COMPONENT TO ULTRASOUND WAVES ⌐S200

CALCULATE TRANSMISSION WAVE FIELD BY FORWARD PROPAGATING ULTRASOUND WAVES TRANSMITTED ON THE BASIS OF SECOND TRANSMISSION CONDITION WHILE IMPLEMENTING SIMULATION METHOD THAT TAKES NON-LINER COMPONENTS OF MEDIUM INTO CONSIDERATION ⌐S300C

CALCULATE RECEPTION WAVE FIELD BY BACKWARD PROPAGATING SIGNALS BASED ON ECHOES ⌐S400

CALCULATE HARMONIC ECHO COMPONENT BY PERFORMING CORRELATION ANALYSIS BETWEEN TRANSMISSION WAVE FIELDS AND RECEPTION WAVE FIELDS ⌐S500

END

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **X. YANG et al.** A reverse time migration-based multistep angular spectrum approach for ultrasonic imaging of specimens with irregular surfaces. *Ultrasonics*, 2020, vol. 108, 106233

- **F. LIN et al.** Ultrasound contrast imaging: influence of scatterer motion in multi-pulse techniques. *IEEE Transactions on Ultrasonics, Ferroelectrics, and Frequency Control*, October 2013, vol. 60 (1)